# FASCICULE DE BREVET EUROPEEN

(11) **EP 3 177 395 B1**
(45) Date de publication et mention de la délivrance du brevet: **30.11.2022**
(21) Numéro de dépôt: 15752971.0
(22) Date de dépôt: 04.08.2015
(51) Int. Cl.: B01J 20/12, B01D 15/18, B01J 20/18, B01J 20/28, B01J 20/30

(54) **ADSORBANTS ZÉOLITHIQUES COMPRENANT UNE ZÉOLITHE À POROSITÉ HIÉRARCHISÉE**
ZEOLITHISCHE ADSORBENTIEN UMFASSEND ZEOLITHE MIT HIERARCHISCHER POROSITÄT
ZEOLITIC ADSORBENTS COMPRISING A ZEOLITE WITH HIERARCHICAL POROSITY

(30) Priorité: 05.08.2014 FR 1457624
(43) Date de publication de la demande: 14.06.2017
(73) Titulaire: IFP Energies nouvelles, 92500 Rueil-Malmaison (FR); ARKEMA FRANCE, 92700 Colombes (FR)
(72) Inventeur: LAROCHE, Catherine, 69390 Vernaison (FR); BOUVIER, Ludivine, 64300 Orthez (FR); LUTZ, Cécile, 64290 Gan (FR); BAUDOT, Arnaud, 69390 Vernaison (FR)
(74) Mandataire: Bandpay & Greuter
(86) Numéro de dépôt international: PCT/EP2015/067967
(87) Numéro de publication internationale: WO 2016/020387

(56) Documents cités:
- WO-A1-2015/032923
- FR-A1- 3 010 328
- FR-A1- 3 010 402
- US-A1- 2011 011 804
- US-A1- 2013 012 377

## Description

L'invention concerne des adsorbants zéolithiques à zéolithe(s) à porosité hiérarchisée utilisables dans le domaine de la séparation des isomères des xylènes, et en particulier la séparation de para-xylène à partir d'un mélange d'hydrocarbures aromatiques en C8 contenant majoritairement les *ortho-, méta-* et para-xylène, l'éthylbenzène, et autres.

Le para-xylène est l'un des grands intermédiaires de la pétrochimie. Il est transformé en anhydride ou en acide téréphtalique ou en méthyltéréphtalate, pour être ensuite polycondensé avec du diéthylèneglycol par exemple. Le polyester PET obtenu est ensuite transformé en fibres synthétiques ou en résines destinées notamment à l'industrie textile, à l'emballage des boissons, et autres.

Le para-xylène est généralement séparé des autres isomères aromatiques en C8 soit par cristallisation soit par chromatographie industrielle connue également sous le nom de séparation par adsorption en lit mobile simulé. La technique de séparation par cristallisation représente aujourd'hui moins de 20% de la production totale de paraxylène, tandis que la séparation par adsorption en lit mobile simulé représente aujourd'hui environ 80% à environ 85% de cette production de para-xylène. Enfin, environ 2% de la production totale de para-xylène provient de la combinaison des deux technologies précitées, où la cristallisation est employée comme étape de finition.

Les techniques de séparation par chromatographie industrielle sont réalisées en phase liquide ou en phase gaz, cependant la séparation par chromatographie industrielle le plus généralement réalisée en phase liquide.

La séparation en lit mobile simulé s'entend ici au sens large, c'est-à-dire qu'on peut avoir affaire soit à un contre-courant simulé, soit à un co-courant simulé, soit à un procédé dit "Varicol". La caractéristique commune à cette famille de procédés est que le solide adsorbant est mis en œuvre en lit fixe, et que les flux, liquides ou éventuellement gazeux en contact avec l'adsorbant, sont gérés soit au moyen d'un jeu de vannes tout ou rien, soit au moyen d'une vanne unique complexe connue sous le nom de vanne rotative ou "rotary valve".

Lorsque l'élément actif des solides adsorbants utilisés comme agents d'adsorption dans ces procédés est une zéolithe, cette dernière, obtenue sous forme de poudre (cristaux), est de préférence employée à l'échelle industrielle sous forme d'agglomérés. Ces adsorbants zéolithiques, agglomérés sous forme de plaquettes, billes ou extrudés, sont en général constitués d'une poudre de zéolithe, qui constitue l'élément actif au sens de l'adsorption et d'un liant destiné à assurer la cohésion des cristaux sous forme de billes ou extrudés, généralement appelés grains.

Ce liant confère également aux grains une résistance mécanique suffisante pour résister aux contraintes mécaniques auxquels ils sont soumis au cours de leur mise en œuvre au sein des unités. Ces contraintes mécaniques sont à l'origine de la formation de fines, qui induisent une détérioration des performances au cours de la durée d'opération du procédé.

Les procédés de séparation des xylènes en lit mobile simulé (LMS) ont connu au cours des dernières décennies de très nombreuses améliorations technologiques, notamment au niveau des plateaux distributeurs des fluides, mais relativement peu d'évolution concernant les caractéristiques granulométriques des particules du solide adsorbant.

Les documents de l'art antérieur décrivant les caractéristiques chimiques et microscopiques des adsorbants zéolithiques utilisés pour la séparation du *para*-xylène sont particulièrement nombreux, et par exemple on peut citer à titre d'illustration les brevets US3558730, US3663638, US3960774, US7820869, US7812208, US8283274, US8530367 et US8735643.

L'enseignement général sur les caractéristiques chimiques de ces solides adsorbants est qu'il faut utiliser une zéolithe de structure faujasite (zéolithe LSX, X ou Y) et dont les ions compensateurs sont majoritairement des ions Ba²⁺ ou majoritairement des ions Ba²⁺ et de manière minoritaire des ions K⁺.

En outre, l'enseignement général sur les caractéristiques microscopiques de l'adsorbant est que l'on peut utiliser des cristaux de zéolithe X de taille de préférence inférieure à 1,6 µm (diamètre moyen en nombre). Quelques documents parmi les plus récents, nous enseignent l'utilisation d'adsorbants à base de cristaux de zéolithe X de taille inférieure à 0,5 µm (US2009/326308) ou comprise entre 0,1 µm et 0,4 µm (CN1267185C), afin d'améliorer les performances du procédé de séparation des isomères du xylène du fait d'un gain en transfert de matière de ces adsorbants par rapport aux adsorbants conventionnels cités plus haut.

Par ailleurs, dans le domaine de la catalyse, notamment dans les procédés d'hydrocraquage, lorsque des supports zéolithiques sont utilisés, il est d'usage de chercher à améliorer l'accessibilité des molécules aux micropores desdites zéolithes en créant des mésopores dans les cristaux de zéolithe FAU (zéolithe Y) par traitement post-synthèse.

Les travaux de Inayat *et coll. (*Angew. Chem. Int. Ed., (2012), 51, 1962-1965) enseignent qu'il est possible de synthétiser des cristaux de zéolithe X mésoporeuse. On peut ainsi s'attendre à ce que l'accessibilité aux micropores soit améliorée par rapport à celle des cristaux de X conventionnels. Par conséquent l'homme du métier serait enclin à vouloir utiliser de tels cristaux de zéolithe X mésoporeuse pour former des adsorbants performants pour la séparation des isomères du xylène.

Toutefois cette publication (Inayat, *ibid*.) montre une perte de 23% du volume microporeux de la zéolithe NaX mésoporeuse synthétisée, par rapport à celui de la zéolithe NaX conventionnelle. Ceci est en totale contradiction avec un volume d'adsorption sélective le plus fort possible qui est recherché pour les adsorbants utilisés en séparation des isomères du xylène.

Force est de constater que, entre gain hypothétique en transfert de matière et perte confirmée de volume microporeux, il est impossible de préjuger des performances d'un adsorbant préparé avec des cristaux de zéolithe X mésoporeuse pour la séparation des isomères du xylène.

En revanche, l'enseignement général concernant les caractéristiques macroscopiques de l'adsorbant est que l'on peut augmenter la teneur en matière active, par transformation du liant en zéolithe sous l'action d'une solution alcaline basique, de sorte que le produit fini contienne une quantité de phase non zéolithique réduite, qui peut être quantifiée par référence à un adsorbant composé uniquement de zéolithe, sous forme de poudre, à partir de mesures d'adsorption ou à partir d'intensités de pics de DRX. Cette transformation du liant en matière active au sens de l'adsorption permet par ailleurs de maintenir la résistance mécanique de l'aggloméré (US8530367), ce qui est nécessaire pour résister aux contraintes mécaniques lors de leur mise en œuvre au sein des unités.

Les documents de l'art antérieur décrivant précisément les caractéristiques granulométriques et morphologiques des adsorbants zéolithiques en liaison avec la technologie des plateaux distributeurs du procédé en lit mobile simulé, sont, quant à eux, beaucoup plus rares et nettement moins précis. Aujourd'hui, l'homme du métier ne semble notamment disposer d'aucun document enseignant comment sélectionner les caractéristiques granulométriques optimales des adsorbants en fonction des propriétés des cristaux de zéolithe X utilisés pour former l'adsorbant.

Certains des documents précédemment cités (par exemple US3960774, US7812208, US2009/326308) mentionnent les techniques usuelles d'agglomération (extrusion éventuellement suivie de concassage, agglomération dans un mélangeur tronconique, par exemple Nautamix^{®}, dans un tambour granulateur, sphéronisateur) permettant d'obtenir des adsorbants sous forme d'extrudés, sphères, billes, dans la classe granulométrique allant de 0,25 mm à 1,2 mm (16-60 Standard US Mesh size) et ce, quel que soit le diamètre des cristaux de zéolithe(s) X utilisée(s) dans l'adsorbant. Dans l'exemple 1 du document US2011/105301, les auteurs divulguent des adsorbants sous forme d'agglomérés, obtenus par agglomération dans un tambour granulateur (« tumbling » en langue anglaise) puis tamisage dans la classe granulométrique allant de 0,35 mm à 0,8 mm.

Dans ces documents, les indications chiffrées sur la granulométrie des adsorbants correspondent en pratique au maillage des deux toiles utilisées pour sélectionner les agglomérés, c'est-à-dire qu'elles correspondent aux bornes inférieures et supérieures du plus petit et du plus grand aggloméré de la distribution, mais ne mentionnent pas de valeurs de diamètre moyen.

Le brevet CN1267185 souligne l'importance d'une distribution granulométrique étroite pour améliorer le remplissage des adsorbants dans les unités industrielles : il divulgue un adsorbant à base de cristaux de X de taille comprise entre 0,1 µm et 0,4 µm, sous forme de particules dont le diamètre est compris entre 0,35 mm et 0,8 mm, et respectant la répartition suivante : 20% en poids à 25% en poids dans la classe granulométrique 0,60 mm à 0,80 mm, 50% en poids à 60% en poids dans la classe granulométrique 0,46 mm à 0,60 mm et 20% en poids à 30% en poids dans la classe granulométrique 0,35 mm à 0,46 mm.

Sachant que les distributions granulométriques des agglomérés obtenus par les techniques standards de mise en forme suivent en général une loi lognormale, une granulométrie distribuée entre 0,25 mm et 1,2 mm (comme par exemple dans US7820869 et US2009/326308) ou entre 0,35 mm et 0,8 mm (par exemple dans US2011/105301) correspond à un diamètre moyen en poids pour une distribution issue d'un tamisage des agglomérés, situé aux alentours des valeurs médianes, à savoir aux alentours de 0,55 mm à 0,65 mm, en fonction de l'écart-type de la distribution.

Le document US8530367 divulgue quant à lui uniquement les diamètres moyens en nombre des adsorbants sous forme de billes ou extrudés, obtenus par extrusion, compactage, ou agglomération, à savoir un diamètre moyen en nombre allant de 0,4 mm à 2 mm et en particulier de 0,4 mm à 0,8 mm, mais aucune mention n'est faite des bornes inférieures et supérieures du plus petit et du plus grand aggloméré de la distribution. Il est simplement précisé que les particules d'agglomérés les plus fines peuvent être éliminées par cyclonage et/ou tamisage et/ou les particules trop grosses par tamisage ou concassage, dans le cas d'extrudés, par exemple.

Il doit être noté qu'un diamètre moyen en nombre de 0,4 mm correspond à un diamètre moyen en volume plus élevé, typiquement de 0,45 mm à 0,55 mm selon l'écart-type de la distribution, et de même un diamètre moyen en nombre de 0,8 mm correspondrait à un diamètre moyen en volume d'environ 0,9 mm à 1,0 mm. Les exemples des documents US7452840 et US8530367 divulguent des agglomérés de diamètre équivalent égal à 0,7 mm obtenus par extrusion, qui sont soumis à concassage et tamisage, aussi bien pour les agglomérés préparés à partir de cristaux de zéolithe X conventionnels de 2,1 µm que pour les agglomérés préparés à partir de cristaux de zéolithe X de diamètre réduit à 1,6 µm. Le document US 2011/011804 A1 divulgue un adsorbant zéolithique sous forme de sphères et le document US2013/012377 A1 divulgue un adsorbant zéolithique sous forme de granules.

Par conséquent, l'art antérieur montre que quelles que soient les propriétés des cristaux de zéolithe(s) X utilisée(s) dans les adsorbants, la granulométrie des adsorbants est inchangée.

Si l'on se réfère à l'expression théorique de la résistance au transfert de matière telle que décrite par Ruthven dans « Principles of Adsorption and Adsorption processes », p 243, (« Principes de l'Adsorption et des procédés d'adsorption »), la taille des adsorbants est un paramètre que l'on chercherait éventuellement à réduire pour favoriser le transfert de matière car la résistance diffusionnelle entre les cristaux (également appelée « résistance macroporeuse ») est proportionnelle au carré des diamètres des adsorbants. Cependant, la réduction de la taille des adsorbants est limitée par l'impact que cela aurait lors de l'utilisation de l'adsorbant dans l'application industrielle, car la granulométrie des adsorbants détermine la perte de charge au sein de l'unité industrielle et l'uniformité du remplissage.

Par ailleurs, il est connu dans la littérature relative aux granulats de béton que pour des mélanges constitués de particules de formes irrégulières et présentant des distributions granulométriques larges, la compacité diminue si la forme des particules s'éloigne de la sphéricité. D'autre part, la compacité augmente avec l'étalement de la distribution granulométrique car les petites particules peuvent se loger dans les interstices créés entre les plus grosses (Cumberland and Crawford, « The Packing of particles », Elsevier, Amsterdam, (1987) ; German, « Introduction to particle packing » in « Powder packing characteristics », (1989) pp 1-20).

La compacité du lit d'adsorbants, que l'on cherche à maximiser pour atteindre une productivité maximale avec un adsorbant donné, dépend également du mode de remplissage. Le chargement de l'adsorbant dans les unités industrielles doit se faire de la manière la plus dense possible de manière à réduire autant que faire se peut la fraction d'espace laissé libre entre les billes (Porosité de lit : εb).

La différence entre un chargement « à la manche » sans aucune précaution et un chargement « dense » atteint facilement 10% en densité de remplissage de lit (grammes d'adsorbant par m³ de lit). Une méthode de choix pour réaliser un chargement dense consiste à créer une « pluie » homogène d'adsorbant sur toute la surface du lit et à laisser le niveau monter suffisamment lentement (plusieurs heures). Il existe différents dispositifs commerciaux (Densicat^{®}, Catapac^{®}, Calipac^{®}) pour ce faire. Le principe consiste à reprendre un écoulement vertical à débit contrôlé d'adsorbant par une série lanières ou de roues horizontales en rotation (à vitesse angulaire bien déterminées) de manière à projeter l'adsorbant depuis le centre vers la périphérie.

La demande WO2008/152319 décrit des agglomérés de taille et de morphologie maitrisées et à très haute sphéricité obtenus par une technique de mise en forme particulièrement intéressante qui est l'atomisation. Ce document montre qu'il est possible d'obtenir par atomisation des billes à la fois suffisamment denses et résistantes mécaniquement et présentant une très bonne sphéricité dans une gamme de taille (diamètre moyen) allant de 50 µm à 600 µm et de préférence de 50 µm à 500 µm. Toutefois, ce document n'enseigne pas comment sélectionner les caractéristiques optimales (taille et morphologie notamment) de l'adsorbant en fonction des propriétés des cristaux de zéolithe.

Il est par ailleurs constant de rechercher à augmenter toujours plus la productivité de séparation de *para*-xylène à partir de coupes aromatiques à 8 atomes de carbone. Pour réaliser cet objectif, l'art antérieur précité enseigne qu'une solution pourrait être d'améliorer le transfert de matière intragranulaire au sein de l'adsorbant, et d'augmenter la quantité d'adsorbant par volume de lit et diminuer la porosité du lit, c'est-à-dire disposer d'un lit d'adsorbant plus compact et plus dense.

D'après l'expression théorique de la résistance au transfert de matière telle que décrite par Ruthven dans « Principles of Adsorption and Adsorption processes », p 243, (« Principes de l'Adsorption et des procédés d'adsorption ») une des solutions pour atteindre ce but serait également de diminuer la taille des billes d'adsorbants zéolithiques. Cependant, un des inconvénients directement lié à la réduction toujours plus importante de la taille des billes est une augmentation conséquente de la perte de charge. Pour compenser l'augmentation de la perte de charge, il est nécessaire d'augmenter la pression qui doit être appliquée sur les lits d'adsorbant et ainsi prendre le risque de briser les adsorbants zéolithiques dans les lits.

Ainsi l'art antérieur prône plus préférentiellement la solution qui consiste à diminuer encore la taille des cristaux de zéolithe au sein d'adsorbants zéolithiques dont la taille (diamètre moyen en volume) de billes n'est pas inférieure à une valeur de l'ordre de 0,5 mm.

Il reste donc un besoin pour des adsorbants zéolithiques sous forme d'agglomérés comprenant des cristaux de zéolithe FAU de type X, dont les ions compensateurs sont majoritairement des ions baryum ou des ions baryum et potassium, permettant encore d'augmenter la productivité en para-xylène, sans augmenter la perte de charge dans des unités de séparation du para-xylène en lit mobile simulé.

Le terme « majoritairement » utilisé ci-dessus pour caractériser les ions compensateurs présents dans la zéolithe de type X, signifie une quantité molaire supérieure à 50% d'ions par rapport à la quantité molaire totale d'ions présents dans les sites cationiques desdites zéolithes de type X. Il doit être compris que tous les sites cationiques desdites zéolithes X sont occupés par un cation compensateur de charge, les zéolithes utilisées étant électroniquement neutres lors de leur utilisation.

Le besoin pour des adsorbants zéolithiques encore plus performants dans les applications de séparation de para-xylène en lit mobile simulé décrit ci-dessus peut encore être exprimé par un besoin en adsorbants zéolithiques qui combineraient les propriétés suivantes :
- volume d'adsorption sélective de l'adsorbant zéolithique le plus grand possible par unité de volume de lit, c'est à dire :
   a) teneur en zéolithe la plus grande possible, la zéolithe constituant la microporosité au sein de laquelle a lieu l'adsorption sélective ;
   β) porosité de grain la plus faible possible (densité de grain élevée)
   γ) porosité du lit d'adsorbant la plus faible possible (compacité élevée)
- transfert de matière au sein de l'adsorbant zéolithique le plus rapide possible, c'est-à-dire temps minimal pour une molécule d'hydrocarbure pour aller de l'extérieur de l'adsorbant au cœur des cristaux de zéolithe de l'adsorbant zéolithique.

Autrement dit encore, il reste un besoin pour des adsorbants zéolithiques combinant à la fois un transfert microporeux optimal et un transfert macroporeux amélioré tout en conservant un volume d'adsorption sélective par unité de volume de lit suffisant de façon à maximiser le gain de productivité lors de leur utilisation dans les procédés de séparation de para-xylène dans des unités de séparation utilisant la technique dite en lit mobile simulé.

Il a maintenant été trouvé que les objectifs exprimés ci-dessus peuvent être atteints, en totalité ou au moins en partie, grâce aux adsorbants zéolithiques selon l'invention décrite dans la description qui suit.

Les inventeurs ont en effet découvert de manière surprenante que l'utilisation d'adsorbants zéolithiques obtenus à partir de zéolithe(s) à porosité hiérarchisée(s) ou zéolithe(s) dite « mésoporeuse(s) » dans la présente invention permet de réduire encore la taille (diamètre moyen en volume) des adsorbants zéolithiques, sans conduire à une augmentation substantielle de la perte de charge, et ce, quelle que soit la taille des cristaux zéolithiques au sein desdits adsorbants et en particulier pour des tailles de cristaux égales à la taille des cristaux conventionnels ou supérieures, c'est-à-dire typiquement de tailles micrométriques c'est-à-dire de l'ordre de grandeur du micromètre.

En d'autres termes, il a été découvert qu'il est possible de s'affranchir de la totalité ou au moins d'une partie des inconvénients relevés dans l'art antérieur en mettant en œuvre un procédé de séparation de para-xylène utilisant un adsorbant zéolithique selon l'invention dont les caractéristiques granulométriques (diamètre moyen en volume, distribution granulométrique et morphologie) sont telles qu'elles permettent de maximiser la compacité de lit. Ces caractéristiques granulométriques conduisent à un transfert macroporeux adapté et optimal.

Ainsi et selon un premier aspect, la présente invention concerne un adsorbant zéolithique pour lequel :
- la teneur en phase cristalline sous forme de zéolithe FAU de type X à porosité hiérarchisée, c'est-à-dire possédant à la fois des micropores et des mésopores identifiables par observation au moyen d'un Microscope Électronique à Transmission, est comprise entre 50% et 99% en poids, de préférence de 80% à 98% en poids, par rapport à la masse totale de l'adsorbant, la teneur en phase cristalline étant mesurée par analyse par diffraction de rayons X ;
- la teneur en ions baryum (Ba²⁺), exprimée en oxyde de baryum (BaO) est supérieure à 10%, de préférence supérieure à 15%, de manière très préférée supérieure à 20%, de manière encore plus préférée supérieure à 23%, voire supérieure à 33% en poids par rapport à la masse totale de l'adsorbant, avantageusement, la teneur en oxyde de baryum est comprise entre 23% et 42%, et typiquement entre 30% et 40%, en poids par rapport au poids total de l'adsorbant, la teneur en ions baryum étant mesurée par analyse chimique par fluorescence de rayons X selon la norme NF EN ISO 12677 : 2011 ;
- la teneur en ions potassium (K⁺), exprimée en oxyde de potassium (K₂O) est inférieure à 25%, de préférence comprise entre 0% et 20%, de manière encore plus préférée comprise entre 0% et 15% et de manière très préférée de 0% à 10% en poids par rapport à la masse totale de l'adsorbant, la teneur en ions potassium étant mesurée par analyse chimique par fluorescence de rayons X selon la norme NF EN ISO 12677 : 2011 ;
- le diamètre moyen en volume est compris entre 0,2 mm et 0,6 mm, et de préférence compris entre 0,3 mm et 0,6 mm, mesuré par analyse de la distribution granulométrique d'un échantillon d'adsorbant par imagerie selon la norme ISO 13322-2:2006 et calculé à partir de la distribution granulométrique en appliquant la norme ISO 9276-2:2001 ; et
   dont les caractéristiques de sphéricité sont telles que :
- l'émoussé moyen est compris entre 70% et 100%, de préférence entre 80% et 100%, de manière préférée entre 85 % et 100%, de manière encore plus préférée entre 90 % et 100%, mesuré par la microscopie électronique à balayage ;
- le pourcentage de particules ayant un allongement de 0% est compris entre 5 et 100, de préférence entre 10 et 100 et de manière préférée entre 25 et 100, mesuré par la microscopie électronique à balayage ; et
- la surface externe, mesurée par adsorption d'azote et calculée par la méthode du t-plot à partir de l'isotherme d'adsorption d'azote à une température de 77K, après dégazage sous vide (P < 6,7*10⁻⁴ Pa), à une température comprise entre 300°C et 450°C pendant une durée allant de 9 heures à 16 heures, en appliquant la norme ISO 15901-3:2007, est supérieure à 20 m².g⁻¹.

Dans un mode de réalisation préféré, l'adsorbant zéolithique de l'invention présente une surface externe, mesurée par adsorption d'azote (méthode du t-plot décrite plus loin), supérieure à 30 m².g⁻¹, et de préférence encore comprise entre 30 m².g⁻¹ et 200 m².g⁻¹, et plus préférentiellement comprise entre 30 m².g⁻¹ et 150 m².g⁻¹.

Selon encore un autre mode de réalisation préféré, l'adsorbant zéolithique de l'invention présente une surface externe, mesurée par adsorption d'azote (méthode du t-plot décrite plus loin), supérieure à 20 m².g⁻¹, de préférence supérieure à 30 m².g⁻¹, et de préférence encore comprise entre 30 m².g⁻¹ et 200 m².g⁻¹, et plus préférentiellement comprise entre 30 m².g⁻¹ et 150 m².g⁻¹, ladite surface externe étant associée à une population de mésopores de diamètre moyen compris entre 2 nm et 50 nm. Ce diamètre étant déterminé par la méthode de Barrett-Joyner-Halenda à partir de la branche d'adsorption de l'isotherme de physisorption d'azote à 77K. Le terme « associée » dans la phrase précédente indique que la population de mésopores contribue à la valeur mesurée de la surface externe, en plus de la surface externe des cristaux de zéolithe(s).

Dans ce qui va suivre, et à moins d'une autre indication, les bornes d'un domaine de valeurs sont comprises dans ce domaine, notamment dans les expressions « compris entre » et « allant de ... à ... ».

Par « zéolithe à porosité hiérarchisée », on entend une zéolithe possédant à la fois des micropores et des mésopores, autrement dit une zéolithe à la fois microporeuse et mésoporeuse. Par « zéolithe mésoporeuse », on entend une zéolithe dont les cristaux zéolithiques microporeux présentent, conjointement à la microporosité, des cavités internes de taille nanométrique (mésoporosité), facilement identifiables par observation au moyen d'un Microscope Électronique à Transmission (MET ou « TEM » en langue anglaise), comme décrit par exemple dans US 7 785 563.

De préférence, dans la présente invention on utilisera une « zéolithe à porosité hiérarchisée » ou « zéolithe mésoporeuse », présentant une surface externe, définie par la méthode du t-plot décrite plus loin, comprise entre 40 m².g⁻¹ et 400 m².g⁻¹, de préférence entre 40 m².g⁻¹ et 200 m².g⁻¹, de préférence encore entre 40 m².g⁻¹ et 150 m².g⁻¹.

Dans la présente invention, les surfaces externes de l'adsorbant zéolithique ou de la zéolithe à porosité hiérarchisée sont calculées par la méthode du t-plot à partir de l'isotherme d'adsorption d'azote à une température de 77K, après dégazage sous vide (P < 6,7.10⁻⁴ Pa), à une température comprise entre 300°C et 450°C pendant une durée allant de 9 heures à 16 heures, de préférence à 400°C pendant 10 heures.

Le diamètre moyen des mésopores est déterminé par la méthode Barrett-Joyner-Halenda (méthode BJH, E. P. Barrett, L. G. Joyner, P. P. Halenda, "The Determination of Pore Volume and Area Distributions in Porous Substances. I. Computations form Nitrogen Isotherms", J. Am. Chem. Soc., 73(1), (1951), 373-380), à partir de la branche d'adsorption de l'isotherme de physisorption d'azote à 77K.

Avantageusement, la distribution en volume de diamètre moyen ainsi déterminé des mésopores de l'adsorbant selon l'invention, représentée graphiquement par *d*V/*d*Dm ou *d*V/*d*logDm en fonction du diamètre moyen Dm, correspond à une distribution unimodale et étroite.

Par « distribution unimodale », on entend une distribution ne présentant qu'un seul pic. Une distribution unimodale de diamètre moyen est ainsi caractérisée par un seul pic, pour lequel la valeur du diamètre moyen au sommet du pic est appelé « mode », ou encore valeur dominante, et représente la valeur la plus fréquente de la distribution. Lorsqu'une distribution présente deux pics séparés par un creux, on dit que la distribution est bimodale. L'invention ne concerne pas le cas de distribution bimodale voire multimodale, c'est-à-dire de distribution où il existe plusieurs zones de concentration des valeurs séparées par des discontinuités. De telles distributions sont caractéristiques de la présence de plusieurs populations de pores de diamètres moyens distincts.

Le terme « étroite », utilisé pour caractériser la distribution de diamètre moyen des mésopores, indique que la largeur à mi-hauteur de la distribution autour du mode est inférieure à 20 nm, de préférence inférieure à 15 nm, de manière préférée comprise entre 10 nm et 0,1 nm et de manière encore préférée comprise entre 5 nm et 0,5 nm, comme décrit plus loin dans les techniques de caractérisation.

Les cristaux de la (ou des) zéolithe(s) mésoporeuse(s) comprise(s) dans l'adsorbant zéolithique de l'invention, seuls ou en mélange avec d'autres cristaux de zéolithes, identiques ou différentes, mésoporeuses ou non mésoporeuses, sont agglomérés avec un liant. Selon un aspect préféré de l'invention, la teneur en liant est la plus faible possible, afin d'optimiser la teneur en zéolithe(s) de l'adsorbant zéolithique, mais cependant suffisante pour assurer la cohésion du matériau.

Le liant compris dans l'adsorbant zéolithique de la présente invention est choisi parmi les liants classiques connus de l'homme du métier, zéolithisables ou non zéolithisables. Ce liant comprend, et de préférence consiste en, une argile ou un mélange d'argiles. Ces argiles sont de préférence choisies parmi les kaolins, kaolinites, nacrites, dickites, halloysites, attapulgites, sépiolites, montmorillonites, bentonites, illites et métakaolins, ainsi que les mélanges de deux ou plusieurs d'entre elles en toutes proportions.

Dans la présente invention, le « liant » décrit ci-dessus est un liant d'agglomération qui permet d'assurer la cohésion des cristaux de zéolithe(s) dans l'adsorbant zéolithique (ou matériau zéolithique aggloméré) de l'invention. Ce liant se distingue en outre des cristaux de zéolithe(s) en ce qu'ils ne présentent pas de structure cristalline zéolithique après calcination, raison pour laquelle le liant est souvent qualifié d'inerte, et plus précisément inerte vis-à-vis de l'adsorption et de l'échange ionique.

Selon un aspect particulièrement préféré, le liant présent dans l'adsorbant zéolithique de l'invention est uniquement constitué d'une ou plusieurs argiles, et de préférence d'une seule argile.

L'adsorbant zéolithique selon la présente invention peut également comprendre un ou plusieurs autres composants, en quantité comprise entre 0 et 5%, de préférence entre 0 et 1%, de préférence encore entre 0 et 0,5%, les pourcentages étant exprimés en poids par rapport au poids total de l'adsorbant zéolithique. Ce ou ces autre(s) composant(s) est(sont) généralement les résidus des additifs, et autres auxiliaires de synthèse dudit adsorbant zéolithique.

Des exemples de tels autres composants comprennent notamment les cendres des additifs après calcination, de la silice, et autres. Il doit être compris que ces autres composants sont généralement présents à l'état de résidus ou de traces et ne sont pas utilisés pour apporter un quelconque caractère liant ou cohésif adsorbants zéolithiques comprenant au moins une zéolithe mésoporeuse de l'invention.

L'adsorbant zéolithique de la présente invention se présenter sous forme de billes.

Selon un mode de réalisation non selon la présente invention, l'adsorbant zéolithique se présente sous forme d'agglomérés zéolithiques comprenant de la zéolithe X à porosité hiérarchisée et dont les ions compensateurs sont majoritairement du baryum ou du baryum et du potassium, lesdits adsorbants présentant des caractéristiques de taille et de morphologie particulières par rapport à un adsorbant conventionnel.

L'expression « majoritairement du baryum ou du baryum et du potassium » signifie que l'adsorbant zéolithique présente :
- une teneur en baryum, exprimée en oxyde de baryum (BaO) supérieure à 10%, de préférence supérieure à 15%, de manière très préférée supérieure à 20%, de manière encore plus préférée supérieure à 23 %, voire supérieure à 33% en poids par rapport à la masse totale de l'adsorbant, avantageusement, la teneur en oxyde de baryum est comprise entre 23% et 42%, et typiquement entre 30% et 40%, en poids par rapport au poids total de l'adsorbant, et
- une teneur en potassium, exprimée en oxyde de potassium (K₂O) inférieure à 25%, de préférence comprise entre 0 et 20%, de manière encore plus préférée comprise entre 0% et 15% et de manière très préférée de 0% à 10% en poids par rapport à la masse totale de l'adsorbant.

Selon encore un autre mode de réalisation préféré, la teneur totale en ions alcalins ou alcalino-terreux, autres que baryum et potassium, exprimée en teneur totale d'oxydes d'ions alcalins ou alcalino-terreux autres que l'oxyde de baryum BaO et l'oxyde de potassium K₂O, est comprise entre 0 et 5%, par rapport à la masse totale de l'adsorbant.

Comme indiqué précédemment l'utilisation de la présente invention met avantageusement en œuvre un adsorbant zéolithique présentant des caractéristiques de taille et de morphologie particulières par rapport à un adsorbant conventionnel.

Il a en effet été découvert de manière surprenante que, par rapport à un aggloméré de diamètre moyen en volume de 0,7 mm comprenant de la zéolithe X à porosité hiérarchisée, on améliore la productivité en sélectionnant une forme de billes dont le diamètre moyen peut être encore réduit sans pour autant augmenter significativement la perte de charge.

Ainsi, outre le diamètre moyen en volume compris entre 0,2 mm et 0,6 mm, et de préférence compris entre 0,3 mm et 0,6 mm, l'adsorbant zéolithique de l'invention se présente sous forme de billes, dont les caractéristiques de sphéricité sont telles que :
- l'émoussé moyen est compris entre 70% et 100%, de préférence entre 80% et 100%, de manière préférée entre 85 % et 100%, de manière encore plus préférée entre 90 % et 100%;
- le pourcentage de particules ayant un allongement de 0% est compris entre 5 et 100, de préférence entre 10 et 100 et de manière préférée entre 25 et 100.

Selon un mode de réalisation préféré, l'adsorbant zéolithique de l'invention présente en outre la caractéristique de sphéricité suivante : le pourcentage de particules ayant un allongement inférieur à 10 % est compris entre 50 et 100 et de préférence entre 70 et 100 et de manière préférée entre 80 et 100.

Les méthodes de mesure de la sphéricité sont bien connues de l'homme du métier et sont décrites plus loin dans les techniques de caractérisation.

L'adsorbant zéolithique de la présente invention présente une surface externe, mesurée par adsorption d'azote, supérieure à 20 m².g⁻¹, de préférence supérieure à 30 m².g⁻¹, et de préférence encore comprise entre 30 m².g⁻¹ et 200 m².g⁻¹, et plus préférentiellement comprise entre 30 m².g⁻¹ et 150 m².g⁻¹.

Selon encore un autre mode de réalisation préféré, l'adsorbant zéolithique selon l'invention présente un rapport atomique Si/AI compris entre 1,00 et 2,00 de préférence entre 1,00 et 1,80, de préférence encore entre 1,15 et 1,80, et de manière encore plus préférée entre 1,15 et 1,60.

L'adsorbant zéolithique de l'invention a de préférence une densité de grain comprise entre 1,0 g.cm⁻³ et 1,4 g.cm⁻³, et de manière préférée entre 1,1 g.cm⁻³ et 1,3 g.cm⁻³ telle que mesurée par intrusion de mercure (exprimée par rapport à la masse sèche de l'adsorbant zéolithique) et un volume total contenu dans les macropores et les mésopores (somme du volume macroporeux et du volume mésoporeux) mesuré par intrusion de mercure, compris entre 0,20 cm³.g⁻¹ et 0,40 cm³.g⁻¹ et de préférence compris entre 0,20 cm³.g⁻¹ et 0,35 cm³.g⁻¹ (exprimé par rapport à la masse équivalent anhydre de l'adsorbant zéolithique).

On préfère également dans le cadre de la présente invention un adsorbant zéolithique dont le volume microporeux évalué par la méthode de t-plot à partir de l'isotherme d'adsorption d'azote (N₂) à une température de 77 K, est compris entre 0,180 cm³.g⁻¹ et 0,290 cm³.g⁻¹, de préférence entre 0,180 cm³.g⁻¹ et 0,270 cm³.g⁻¹. Ladite mesure de volume microporeux est calculée après dégazage sous vide (P < 6,7.10⁻⁴ Pa), à une température comprise entre 300°C et 450°C pendant une durée allant de 9 heures à 16 heures, de préférence à 400°C pendant 10 heures.

En outre, l'adsorbant zéolithique de la présente invention présente avantageusement un ratio (volume macroporeux)/(volume macroporeux + volume mésoporeux) compris entre 0,2 et 1, de manière préférée compris entre 0,4 et 0,8, et de manière encore plus préférée entre 0,45 et 0,65.

Dans le cadre de la présente invention, la résistance mécanique est mesurée par la méthode Shell série SMS1471-74 adaptée pour des agglomérés de taille inférieure à 1,6 mm. Cette résistance mécanique, mesurée pour l'adsorbant zéolithique défini précédemment, est généralement comprise entre 1 MPa et 4 MPa, de préférence entre 1,5 MPa et 4 MPa de préférence encore entre 1,8 MPa et 4 MPa et de manière tout à fait préférée entre 2 MPa et 4 MPa.

Un autre objet non selon l'invention concerne le procédé de préparation de l'adsorbant zéolithique tel que défini précédemment, ledit procédé comprenant au moins les étapes de :
a) agglomération de cristaux d'au moins une zéolithe de type FAU à porosité hiérarchisée, présentant une surface externe comprise entre 40 m².g⁻¹ et 400 m².g⁻¹, de préférence comprise entre 40 m².g⁻¹ et 200 m².g⁻¹, de préférence encore comprise entre 40 m².g⁻¹ et 150 m².g⁻¹ avec un liant comprenant au moins 80% d'argile ou d'un mélange d'argiles, éventuellement zéolithisable(s), et éventuellement jusqu'à 5% d'additifs ainsi qu'avec la quantité d'eau qui permet la mise en forme du matériau aggloméré ; séchage des agglomérats à une température comprise entre 50°C et 150°C ; calcination des agglomérats séchés sous balayage gazeux oxydant et/ou inerte, avec notamment des gaz tels que l'oxygène, l'azote, l'air, un air sec et/ou décarbonaté, un air appauvri en oxygène, éventuellement sec et/ou décarbonaté, à une température supérieure à 150°C, typiquement comprise entre 180°C et 800°C, préférentiellement entre 200°C et 650°C, pendant quelques heures, par exemple de 2 à 6 heures ;
b) éventuellement zéolithisation de tout ou partie du liant par mise en contact des agglomérats obtenus à l'étape a) avec une solution basique alcaline ;
c) échange(s) cationique(s) des agglomérats de l'étape a) et/ou de l'étape b) par mise en contact avec une solution d'ions baryum et/ou d'ions potassium ;
d) échange cationique éventuel supplémentaire des agglomérats de l'étape c) par mise en contact avec une solution d'ions potassium ;
e) lavage et séchage des agglomérats obtenus aux étapes c) ou d), à une température comprise entre 50°C et 150°C ; et
f) obtention de l'adsorbant zéolithique selon l'invention par activation des agglomérats obtenus à l'étape e) sous balayage gazeux oxydant et/ou inerte, avec notamment des gaz tels que l'oxygène, l'azote, l'air, un air sec et/ou décarbonaté, un air appauvri en oxygène, éventuellement sec et/ou décarbonaté, à une température comprise entre 100°C et 400°C, de préférence entre 200°C et 300°C pendant une durée déterminée en fonction de la teneur en eau et de la perte au feu souhaitées, typiquement de 1 à 6 heures.

Selon un mode de réalisation préféré, ladite au moins une zéolithe FAU présente un rapport atomique Si/AI compris de préférence entre 1,00 et 1,50, de préférence entre 1,05 et 1,40 et de manière encore plus préférée compris entre 1,10 et 1,40. Comme indiqué précédemment, la surface externe des cristaux mis en œuvre dans l'étape a) du procédé décrit ci-dessus est calculée par la méthode du t-plot à partir de l'isotherme d'adsorption d'azote à une température de 77K, après dégazage sous vide (P < 6,7.10⁻⁴ Pa), à une température comprise entre 300°C et 450°C pendant une durée allant de 9 heures à 16 heures, de préférence à 400°C pendant 10 heures.

De préférence, les cristaux de la zéolithe FAU de l'adsorbant de la présente invention, et avantageusement mis en œuvre à l'étape a) du procédé précédemment défini, présentent un diamètre moyen en nombre compris entre 0,1 µm et 20 µm, de préférence entre 0,5 µm et 20 µm, de préférence encore entre 0,5 µm et 10 µm, de manière préférée entre 0,5 µm et 5 µm.

Les cristaux de la zéolithe FAU présentant une importante surface externe mise en œuvre à l'étape a) peuvent être obtenus selon diverses méthodes connues de l'homme du métier et par exemple selon la synthèse décrite par Inayat et coll. dans Angew. Chem. Int. Ed., 51, (2012), 1962-1965.

Il est également possible de préparer lesdits cristaux par synthèse par ensemencement et/ou par ajustement des conditions opératoires de synthèse tels que le rapport SiO₂/Al₂O₃, la teneur en sodium et l'alcalinité du mélange de synthèse ou encore selon des procédés de post-traitement de cristaux de zéolithe FAU conventionnels.

Les procédés de post-traitements consistent généralement à éliminer des atomes du réseau zéolithique déjà formé, soit par un ou plusieurs traitements acides qui désaluminent le solide, traitement(s) suivi(s) par un ou plusieurs lavage(s) à la soude afin d'éliminer les résidus aluminiques formés, comme décrit par exemple par D. Verboekend et coll. (Adv. Funct. Mater., 22, (2012), pp. 916-928), soit encore par des traitements qui associent l'action d'un acide et celle d'un agent structurant qui améliorent l'efficacité du traitement acide, comme décrit par exemple dans la demande WO 2013/106816.

Les procédés de synthèse directe de ces zéolithes (c'est-à-dire des procédés de synthèse autre que le post-traitement) font généralement intervenir un ou plusieurs agents structurants ou gabarits sacrificiels.

Les gabarits sacrificiels pouvant être utilisés peuvent être de tout type connu de l'homme du métier et notamment ceux décrits dans la demande WO2007/043731. Selon un mode de réalisation préféré, le gabarit sacrificiel est avantageusement choisi parmi les organosilanes et plus préférentiellement parmi le chlorure de [3-(triméthoxy-silyl)propyl]octadecyldiméthylammonium, le chlorure de [3-(triméthoxysilyl)propyl]hexadécyldiméthylammonium, le chlorure de [3-(triméthoxysilyl)propyl]dodécyldiméthylammonium, le chlorure de [3-(triméthoxysilyl)propyl]octylammonium, la N-[3-(triméthoxysilyl)propyl]aniline, le 3-[2-(2-aminoéthylamino)éthylamino]propyltriméthoxysilane, la N-[3-(triméthoxysilyl)propyl]-N'-(4-vinylbenzyl)éthylènediamine, le triéthoxy-3-(2-imidazolin-1-yl)propylsilane, la 1-[3-(triméthoxysilyl)propyl]urée, la N-[3-(triméthoxysilyl)propyl]éthylènediamine, le [3-(diéthylamino)propyl]triméthoxysilane, le (3-glycidyloxypropyl)triméthoxysilane, le méthacrylate de 3-(triméthoxysilyl)propyle, le [2-(cyclohexényl)éthyl]triéthoxysilane, le dodécyltriéthoxysilane, l'hexadécyltriméthoxysilane, le (3-aminopropyl)tri-méthoxysilane, le (3-mercaptopropyl)triméthoxysilane, le (3-chloropropyl)triméthoxysilane, ainsi que les mélanges de deux ou plusieurs d'entre eux en toutes proportions.

Parmi les gabarits sacrificiels listés ci-dessus, on préfère tout particulièrement le chlorure de [3-(triméthoxysilyl)propyl]octadecyldiméthylammonium, ou TPOAC.
On peut également utiliser des gabarits sacrificiels de masse molaire plus élevée et par exemple les PPDA (Polymer Poly-DiallyldimethylAmmonium), PVB (PolyVinyl Butyral) et autres composés oligomères connus dans le domaine pour augmenter le diamètre des mésopores.

Selon un mode de réalisation préféré du procédé de la présente invention, on procède, à l'étape a), à l'agglomération de cristaux d'au moins une zéolithe FAU à porosité hiérarchisée, comme décrit précédemment, préparée en présence d'un gabarit sacrificiel destiné à être éliminé.

Cette élimination peut être réalisée selon les méthodes connues de l'homme du métier, par exemple par calcination, et de manière non limitative, la calcination des cristaux de zéolithe comprenant le gabarit sacrificiel peut être effectuée sous balayage gazeux oxydant et/ou inerte, avec notamment des gaz tels que l'oxygène, l'azote, l'air, un air sec et/ou décarbonaté, un air appauvri en oxygène, éventuellement sec et/ou décarbonaté, à une ou des températures supérieures à 150°C, typiquement comprises entre 180°C et 800°C, préférentiellement entre 200°C et 650°C, pendant quelques heures, par exemple entre 2 et 6 heures. La nature des gaz, les rampes de montée en température et les paliers successifs de températures, leurs durées seront adaptées en fonction de la nature du gabarit sacrificiel.

L'étape supplémentaire d'élimination de l'éventuel gabarit sacrificiel peut être effectuée à tout moment au cours du procédé de préparation du matériau zéolithique aggloméré de l'invention. L'élimination dudit gabarit sacrificiel peut ainsi avantageusement être effectuée par calcination des cristaux de zéolithe avant l'étape d'agglomération a), ou encore de manière concomitante avec la calcination des agglomérés lors de l'étape a).

On ne sortirait pas du cadre de l'invention si l'agglomération de l'étape a) comprenait l'agglomération de plusieurs zéolithes à porosité hiérarchisée obtenues selon des modes différents.

La synthèse de zéolithe de type FAU se fait généralement en milieu sodique (cation Na). Les cristaux de zéolithe FAU ainsi obtenus comprennent majoritairement, voir exclusivement des cations sodium. On ne sortirait toutefois pas du cadre de l'invention en utilisant des cristaux ayant subi un ou plusieurs échanges cationiques, entre la synthèse sous forme Na, avant ou après l'élimination éventuelle du gabarit sacrificiel si cette étape est opérée avant la mise en œuvre à l'étape a) et sa mise en œuvre à l'étape a). Dans ce cas, l'étape c) et éventuellement l'étape d) d'échange devien(nen)t par conséquent non nécessaire(s).

La taille des cristaux de zéolithe FAU utilisés à l'étape a) et des cristaux de zéolithe FAU dans les agglomérés selon l'invention est mesurée par observation au microscope électronique à balayage (MEB). Comme indiqué précédemment, de manière préférée, le diamètre moyen des éléments est compris entre 0,1 µm et 20 µm. Cette observation MEB permet également de confirmer la présence de phase non zéolithique comprenant par exemple du liant résiduel (non converti lors de l'étape de zéolithisation) ou toute autre phase amorphe dans les agglomérés.

Dans le présent document, on emploie l'appellation « diamètre moyen en nombre » ou bien « taille », notamment pour les cristaux de zéolithe. La méthode de mesure de ces grandeurs est explicitée plus loin dans la description.

L'agglomération et la mise en forme (étape a) peuvent être réalisées selon toutes les techniques connues de l'homme de l'art, telles qu'extrusion, compactage, agglomération sur assiette granulatrice, tambour granulateur, atomisation et autres, et de préférence par agglomération sur assiette granulatrice, tambour granulateur, atomisation et autres.

Les proportions de liant d'agglomération (voir définition plus loin) et de zéolithe mises en œuvre sont typiquement celles de l'art antérieur, c'est-à-dire de 1% à 50% en poids, par rapport au poids total (liant + zéolithe). Les agglomérés issus de l'étape a), qu'ils soient sous forme de billes, d'extrudés ou autres, ont de préférence un diamètre moyen en volume compris entre 0,2 mm et 0,6 mm, de préférence entre 0,3 mm et 0,6 mm.

À l'issue de l'étape a) les agglomérés les plus fins peuvent être éliminés par cyclonage et/ou tamisage et/ou les agglomérés trop gros par tamisage ou concassage, dans le cas d'extrudés, par exemple.

Le liant compris dans le matériau aggloméré zéolithique de la présente invention comprend, et de préférence consiste en, une argile ou un mélange d'argiles. Ces argiles sont de préférence choisies parmi les kaolins, kaolinites, nacrites, dickites, halloysites, attapulgites, sépiolites, montmorillonites, bentonites, illites et métakaolins, ainsi que les mélanges de deux ou plusieurs d'entre elles en toutes proportions.

Dans le cas de l'étape optionnelle b) de zéolithisation le liant d'agglomération mis en œuvre à l'étape a) contient au moins 80 % de préférence, au moins 90%, de préférence encore au moins 95%, plus particulièrement au moins 96%, en poids, d'au moins une argile zéolithisable et peut également contenir d'autres liants minéraux tels que bentonite, attapulgite, et autres. Par argile zéolithisable, on entend une argile ou un mélange d'argiles qui sont susceptibles d'être converties en matière zéolithique, le plus souvent par action d'une solution basique alcaline. L'argile zéolithisable appartient en général à la famille des kaolins, kaolinites, nacrites, dickites, halloysite et/ou métakaolins. Le kaolin est préféré et le plus couramment utilisé.

Parmi les additifs éventuellement mis en œuvre à l'étape a), on peut trouver une source de silice de tout type connu de l'homme du métier, spécialiste de la synthèse de zéolithes, par exemple de la silice colloïdale, des diatomées, de la perlite, des cendres de calcination (« fly ash » en langue anglaise), du sable, ou toute autre forme de silice solide.

Lors de l'étape a), outre les cristaux de zéolithe FAU et le liant, d'autres additifs peuvent également être ajoutés, par exemple des additifs destinés à faciliter l'agglomération ou à améliorer le durcissement des agglomérés formés tels que la lignine, l'amidon, la carboxyméthylcellulose, et autres additifs connus de l'homme du métier.

Pour la calcination comprise dans l'étape a), la nature des gaz, les rampes de montée en température et les paliers successifs de températures, ainsi que leurs durées respectives, seront adaptés en fonction de la nature du gabarit sacrificiel à éliminer et en fonction de la nature du liant mis en œuvre à l'étape d'agglomération a).

En particulier si le liant d'agglomération contient une ou plusieurs argiles zéolithisables, la calcination permet de transformer l'argile zéolithisable, typiquement le kaolin, en méta-kaolin qui peut après être converti en zéolithe lors de l'étape de zéolithisation (étape b)). Le principe en est exposé dans « Zeolite Molecular Sieves » de D. W. Breck, John Wiley and Sons, New York, (1973), p. 314-315.

La zéolithisation du liant d'agglomération est pratiquée selon toute méthode maintenant bien connue de l'homme du métier et peut par exemple être réalisée par immersion du produit issu de l'étape a) dans une solution basique alcaline, en général aqueuse, par exemple une solution aqueuse d'hydroxyde de sodium et/ou d'hydroxyde de potassium.

En règle générale, la concentration de la solution alcaline de zéolithisation est de préférence comprise entre 0,5 M et 5 M. La zéolithisation s'opère de préférence à chaud, à une température supérieure à la température ambiante, et typiquement à des températures de l'ordre de 80°C à 100°C, par exemple comprises entre la température ambiante (soit environ 20°C) et la température d'ébullition de la solution alcaline de zéolithisation. La durée du processus de zéolithisation est généralement comprise entre quelques dizaines de minutes et quelques heures, de préférence entre environ 1 heure et 8 heures.

Les étapes d'échange(s) cationique(s) c) et d) s'effectuent selon les méthodes classiques connues de l'homme du métier, et le plus souvent par mise en contact des agglomérés issus de l'étape a) avec un sel de baryum et/ou de potassium, tel que le chlorure de baryum (BaCl₂) et/ou de potassium (KCI), en solution aqueuse à une température comprise entre la température ambiante et 100°C, et de préférence comprise entre 80°C et 100°C afin d'obtenir rapidement des teneurs en oxyde de baryum élevées, i.e. de préférence supérieure à 10%, de préférence supérieure à 15%, de manière très préférée supérieure à 20%, de manière encore plus préférée supérieure à 23 %, voire supérieure à 33% en poids par rapport à la masse totale de l'adsorbant.

Avantageusement, la teneur en baryum (exprimée en oxyde de baryum BaO) est comprise entre 23% et 42%, et typiquement entre 30% et 40%, en poids par rapport au poids total de l'adsorbant. On préfère opérer avec un large excès d'ions baryum par rapport aux cations de la zéolithe que l'on souhaite échanger, typiquement un excès de l'ordre de 10 à 12, avantageusement en procédant par échanges successifs.

L'échange éventuel au potassium (étape d) peut être pratiqué avant et/ou après l'échange au baryum (étape c). Comme indiqué précédemment, il est également possible d'agglomérer à l'étape a) des cristaux de zéolithe FAU contenant déjà des ions baryum ou potassium ou baryum et potassium (pré-échange des cations présents dans la zéolithe de type FAU de départ, typiquement cations sodium, par des ions baryum ou potassium ou baryum et potassium avant l'étape a) et s'affranchir (ou non) des étapes c) et/ou d).

De manière surprenante, la demanderesse a observé que l'étape d'échange cationique, qui peut-être délicate en raison de la relative fragilité de la structure des cristaux zéolithiques n'affecte pas les propriétés intrinsèques de surface externe et du volume microporeux (ramené à la masse de l'adsorbant une fois échangé) desdits cristaux zéolithiques.

Après la ou les étape(s) d'échange(s) cationique(s), on procède ensuite à un lavage, généralement et de préférence à l'eau, puis d'un séchage de l'aggloméré ainsi obtenu.

L'activation qui suit le séchage, est conduite de manière classique, selon les méthodes connues de l'homme du métier, par exemple à une température en général comprise entre 100°C et 400°C, de préférence entre 200°C et 300°C pendant une durée déterminée en fonction de la teneur en eau et de la perte au feu souhaitées, typiquement de 1 à 6 heures.

L'adsorbant zéolithique décrit ci-dessus présente des propriétés tout à fait adaptées et avantageuses pour des applications où sont couramment utilisés des tamis moléculaires, et notamment dans les procédés de séparation, et tout particulièrement les procédés industriels de séparation d'isomères.

Ainsi et selon un troisième aspect, la présente invention concerne l'utilisation d'au moins un adsorbant zéolithique selon l'invention et tel qu'il vient d'être défini, pour la séparation de para-xylène à partir de coupes aromatiques à 8 atomes de carbone.

L'utilisation selon la présente invention qui met en œuvre un adsorbant zéolithique comprenant au moins de la zéolithe de type Ba(K)XPH (pour zéolithe X à porosité hiérarchisée, échangée au baryum ou au baryum et au potassium) permet de proposer un procédé de séparation de para-xylène à partir d'un mélange d'hydrocarbures aromatiques en C8 dont la productivité est encore améliorée par rapport à l'art antérieur.

Ainsi, et selon encore un autre aspect, la présente divulgation concerne un procédé de séparation de para-xylène à partir d'un mélange d'hydrocarbures aromatiques en C8, au moyen d'au moins un adsorbant zéolithique tel qu'il vient d'être défini.

Il a été découvert de manière tout à fait surprenante que le procédé mettant en œuvre au moins un adsorbant zéolithique comprenant au moins une zéolithe Ba(K)XPH permet d'obtenir une productivité en para-xylène encore améliorée par rapport à l'art antérieur.

Plus précisément, la présente divulgation concerne un procédé de séparation de para-xylène à partir d'un mélange d'hydrocarbures aromatiques en C8 dont la productivité est améliorée, l'amélioration étant obtenue par l'emploi de l'adsorbant zéolithique défini précédemment et qui comprend de la zéolithe X à porosité hiérarchisée et dont les ions compensateurs sont majoritairement du baryum ou du baryum et du potassium, lesdits adsorbants présentant des caractéristiques de taille et de morphologie particulières par rapport à un adsorbant conventionnel.

Plus particulièrement, l'invention concerne un procédé de séparation de *para*-xylène à partir de coupes d'isomères C₈ aromatiques consistant à utiliser comme agent d'adsorption du *para*-xylène un adsorbant zéolithique tel que défini précédemment, ledit procédé étant mis en œuvre dans des procédés en phase liquide mais aussi en phase gazeuse.

Dans un mode de réalisation, l'invention concerne un procédé de production de *para*-xylène de haute pureté à partir d'une charge d'hydrocarbures aromatiques contenant des isomères à 8 atomes de carbones comprenant au moins les étapes suivantes :
a) mise en contact, dans des conditions d'adsorption adéquates, de la charge avec un lit d'adsorbant zéolithique tel que défini précédemment, de manière à adsorber préférentiellement le para-xylène,
b) mise en contact, dans des conditions de désorption, du lit d'adsorbant avec un désorbant, qui est préférentiellement soit du toluène, soit du *para*-diéthylbenzène,
c) soutirage du lit d'adsorbant d'un flux contenant le désorbant et les produits de la charge les moins sélectivement adsorbés,
d) soutirage du lit d'adsorbant d'un flux contenant le désorbant et le *para*-xylène,
e) séparation du flux issu de l'étape c) en un premier flux contenant le désorbant et un second flux contenant les produits de la charge les moins sélectivement adsorbés, et
f) une séparation du flux issu de l'étape d) en un premier flux contenant le désorbant et un second flux contenant du *para*-xylène à un niveau de pureté supérieure ou égale à 75% et de préférence supérieure ou égale à 99,7%, et de manière préférée d'au moins 99,8%.

Le procédé peut également optionnellement inclure les étapes suivantes :
g) une étape de cristallisation dans un cristalliseur consistant en la cristallisation du *para*-xylène issu de l'étape f), permettant d'obtenir d'une part des cristaux de *para-*xylène imbibés de leur liqueur mère, et d'autre part une liqueur mère qui peut être en partie, voir en totalité, recyclée en mélange avec la charge fraîche à l'entrée de l'unité d'adsorption en lit mobile simulé, et
h) une étape de lavage des cristaux issus de l'étape g) à l'issue de laquelle on récupère du *para-*xylène à une pureté d'au moins 99,7%, et de manière préférée d'au moins 99,8%.

On peut ainsi séparer le produit désiré par chromatographie liquide d'adsorption préparative (en « batch »), avantageusement en lit mobile simulé, c'est-à-dire à contre-courant simulé ou à co-courant simulé, et plus particulièrement à contre-courant simulé.

La séparation chromatographique en lit mobile simulé à contre-courant simulé est bien connue dans l'état de la technique. En règle générale, une unité de séparation en lit mobile simulé comprend au moins une colonne d'adsorption contenant une pluralité de lits d'un adsorbant, interconnectés en boucle fermée. L'unité de séparation en lit mobile simulé comporte au moins trois zones chromatographiques, et éventuellement quatre ou cinq, chacune de ces zones étant constituée par au moins un lit ou une portion de colonne et comprise entre deux points successifs d'alimentation ou soutirage.

Typiquement, on alimente au moins une charge à fractionner et un désorbant (parfois appelé éluant) et l'on soutire au moins un raffinat et un extrait. Les points d'alimentation et de soutirage sont modifiés au cours du temps, typiquement décalés vers le bas d'un lit, et ce, de façon synchrone, ou éventuellement asynchrone lors d'un fonctionnement de type « Varicol »

Par définition, on désigne chacune des zones de fonctionnement par un numéro :
- Zone 1 = zone de désorption du produit recherché (contenu dans l'extrait) comprise entre l'injection du désorbant et le prélèvement de l'extrait ;
- Zone 2 = zone de désorption des composés du raffinat, comprise entre le prélèvement de l'extrait et l'injection de la charge à fractionner ;
- Zone 3 = zone d'adsorption du produit recherché, comprise entre l'injection de la charge et le soutirage du raffinat ; et
- Zone 4 située entre le soutirage de raffinat et l'injection du désorbant.

Les conditions opératoires d'une unité industrielle d'adsorption de type contre-courant simulé sont en général les suivantes :
- nombre de lits : 4 à 24 ;
- nombre de zones : au moins 4 ;
- température : 100 à 250°C, de préférence 150 à 190°C ;
- pression comprise entre la pression de bulle des xylènes à la température du procédé et 3 MPa ;
- rapport des débits désorbant sur charge : 0,7 à 2,5 (par exemple 0,9 à 1,8 pour une unité d'adsorption seule (« stand alone » en langue anglaise) et 0,7 à 1,4 pour une unité d'adsorption combinée à une unité de cristallisation) ;
- taux de recyclage : 2 à 12, de préférence 2,5 à 5.
- temps de cycle, correspondant au temps entre deux injections de désorbant sur un lit donné : de manière avantageuse, compris entre 4 et 18 min.

On pourra en outre se référer à l'enseignement des brevets US2985589, US5284992 et US5629467.

Les conditions opératoires d'une unité industrielle d'adsorption à co-courant simulé sont en général les mêmes que celles fonctionnant à contre-courant simulé à l'exception du taux de recyclage qui est en général compris entre 0,8 et 7. On pourra se référer aux brevets US4402832 et US4498991.

Le solvant de désorption peut être un désorbant dont le point d'ébullition est inférieur à celui de la charge, tel que le toluène mais aussi un désorbant dont le point d'ébullition est supérieur à celui de la charge, tel que le para-diéthylbenzène (PDEB). La sélectivité des adsorbants selon l'invention pour l'adsorption du para-xylène contenu dans des coupes aromatiques en C₈ est optimale lorsque leur perte au feu mesurée à 900°C est comprise en général entre 4,0% et 8,0%, de préférence entre 4,7% et 6,7%. De l'eau et un peu de dioxyde de carbone entrent dans la perte au feu.

La teneur en eau dans les effluents hydrocarbonés est préférentiellement ajustée entre 20 ppm et 150 ppm pour une température du procédé de 165°C à 185°C, en ajoutant de l'eau dans la charge comprenant les coupes d'isomères d'hydrocarbures aromatiques contenant 8 atomes de carbone et/ou dans le désorbant, et ce afin d'obtenir des résultats optimum en productivité.

De manière plus précise l'objet de l'invention est d'optimiser le solide adsorbant mis en œuvre dans le procédé de séparation du *para*-xylène par adsorption en lit mobile simulé, pour maximiser la performance de ce procédé. En général, les performances recherchées pour la séparation d'une charge contenant des xylènes, sont une productivité maximale pour une pureté du produit recherché dans le flux d'extrait au moins égale à 99,7% et même 99,8%, voire 99,9%, et un rendement global du produit recherché au moins égal à 90%, voire supérieur à 95% et de façon préférée supérieur à 97%, voire encore supérieur à 98%.

La présente invention est maintenant décrite à l'aide des exemples qui suivent, qui ont pour but d'illustrer certains modes de réalisation de l'invention, telle qu'elle est revendiquée dans les revendications annexées.

### TECHNIQUES DE CARACTÉRISATION

### Granulométrie des cristaux zéolithiques - Détection des mésopores

L'estimation du diamètre moyen en nombre des cristaux de zéolithe FAU contenus dans les adsorbants zéolithiques selon l'invention est réalisée par observation au microscope électronique à balayage (MEB).

Afin d'estimer la taille des cristaux de zéolithe dans les adsorbants, on effectue un ensemble de clichés à un grossissement d'au moins 5000. On mesure ensuite le diamètre d'au moins 200 cristaux à l'aide d'un logiciel dédié. La précision est de l'ordre de 3%.

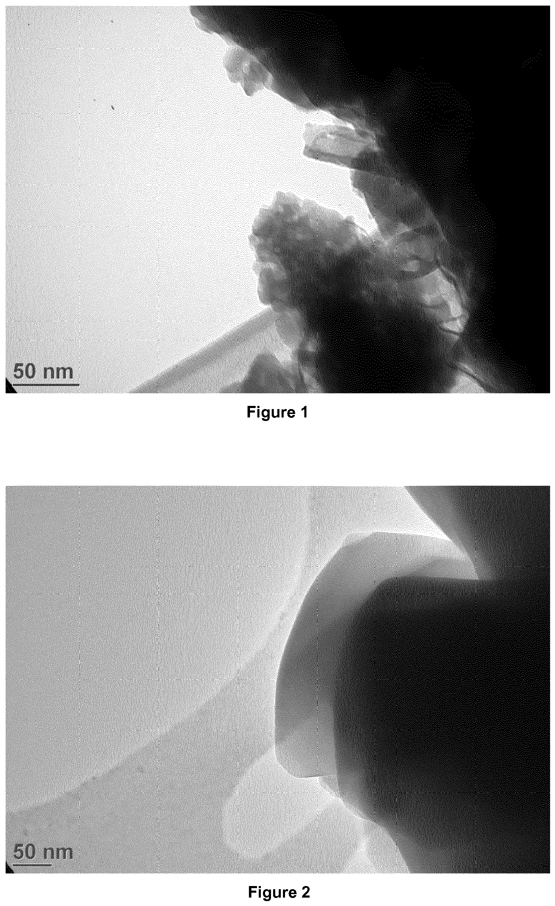

Comme indiqué dans US 7785563, le MET permet en outre de vérifier si les cristaux zéolithiques contenus dans l'adsorbant sont des cristaux de zéolithe pleins (i.e. non mésoporeux) ou des agrégats de cristaux de zéolithes pleins ou des cristaux mésoporeux (cf. la comparaison des clichés MET de la Figure 1, où la mésoporosité est clairement visible et la Figure 2 qui montrent des cristaux pleins). L'observation MET permet ainsi de visualiser la présence ou l'absence des mésopores. De manière préférée, les adsorbants du procédé selon l'invention contiennent très majoritairement, i.e. typiquement plus de 80% et de préférence plus de 90% en nombre des cristaux zéolithiques mésoporeux et non des cristaux pleins. Cette analyse statistique est effectuée avantageusement par analyse d'au moins 50 clichés MET ou MEB (MEB sur sections d'échantillons obtenues par polissage ionique).

### Analyse chimique de l'adsorbant zéolithique - Rapport Si/AI et taux d'échange

Une analyse chimique élémentaire de l'adsorbant zéolithique peut être réalisée selon différentes techniques analytiques connues de l'homme du métier. Parmi ces techniques, on peut citer la technique d'analyse chimique par fluorescence de rayons X telle que décrite dans la norme NF EN ISO 12677 : 2011 sur un spectromètre dispersif en longueur d'onde (WDXRF), par exemple Tiger S8 de la société Bruker.

La fluorescence X est une technique spectrale non destructive exploitant la photoluminescence des atomes dans le domaine des rayons X, pour établir la composition élémentaire d'un échantillon. L'excitation des atomes généralement par un faisceau de rayons X ou par bombardement avec des électrons, génère des radiations spécifiques après retour à l'état fondamental de l'atome. Le spectre de fluorescence X a l'avantage de dépendre très peu de la combinaison chimique de l'élément, ce qui offre une détermination précise, à la fois quantitative et qualitative. On obtient de manière classique après étalonnage pour chaque oxyde, typiquement BaO, Na₂O et K₂O, une incertitude de mesure inférieure à 0,4% en poids.

Ces analyses chimiques élémentaires permettent à la fois de vérifier le rapport atomique Si/AI de l'adsorbant zéolithique et de la zéolithe utilisée lors de la préparation dudit adsorbant, et de vérifier la qualité de l'échange ionique. Dans la description de la présente invention, l'incertitude de mesure du rapport atomique Si/AI est de ± 5%.

La qualité de l'échange ionique est liée au nombre de mole d'oxyde de sodium, Na₂O, restant dans l'aggloméré zéolithique après échange. Plus précisément, le taux d'échange par les ions baryum est estimé en évaluant le rapport entre le nombre de moles d'oxyde de baryum, BaO, et le nombre de moles de l'ensemble (BaO + Na₂O). De même, le taux d'échange par les ions baryum et/ou potassium est estimé en évaluant le rapport entre le nombre de moles de l'ensemble oxyde de baryum + oxyde de potassium (BaO + K₂O) et le nombre de moles de l'ensemble (BaO + K₂O + Na₂O). Il est à noter que les teneurs en différents oxydes sont donnés, en pourcentage en poids par rapport au poids total de l'adsorbant zéolithique anhydre.

### Quantité massique des fractions zéolithiques des adsorbants zéolithiques

La quantité massique des fractions zéolithiques (teneur en phase cristalline) est mesurée par analyse par diffraction de rayons X, connue de l'homme du métier sous l'acronyme DRX. Cette analyse est réalisée sur un appareil de la marque Bruker, puis la quantité des fractions zéolithiques est évaluée à partir des intensités de pic des diffractogrammes en prenant comme référence les intensités de pic d'une référence appropriée (zéolithe de même nature chimique supposée 100% cristalline dans des conditions de traitements cationiques identiques à celles l'adsorbant considéré). Les pics, permettant de remonter à la teneur en phase cristalline, sont les pics les plus intenses de la zone angulaire 2θ comprise entre 9° et 37°, à savoir les pics observés dans les plages angulaires 2θ comprises respectivement entre 11° et 13°, entre 22° et 26° et entre 31° et 33°.

### Granulométrie des adsorbants zéolithiques

La détermination du diamètre moyen en volume des adsorbants zéolithiques du procédé selon l'invention est effectuée par analyse de la distribution granulométrique d'un échantillon d'adsorbant par imagerie selon la norme ISO 13322-2:2006, en utilisant un tapis roulant permettant à l'échantillon de passer devant l'objectif de la caméra.

Le diamètre moyen en volume est ensuite calculé à partir de la distribution granulométrique en appliquant la norme ISO 9276-2:2001. Dans le présent document, on emploie l'appellation « diamètre moyen en volume » ou bien « taille » pour les adsorbants zéolithiques. La précision est de l'ordre de 0,01 mm pour la gamme de taille des adsorbants utiles dans le cadre de la présente invention.

### Facteur de forme des adsorbants zéolithiques

La microscopie électronique à balayage permet une observation et une appréciation visuelle de la morphologie des adsorbants zéolithiques. La morphométrie, basée sur l'acquisition vidéo et sur l'analyse d'images, permet d'accéder à des paramètres quantifiables caractéristiques de la morphologie des particules. Il existe différents dispositifs commerciaux : à titre d'exemples, on peut citer les appareils Morphologi G2 de Malvern, Camsizer de Retsch, Alpaga 500 Nano de Occhio, décrits sur les pages internet www.malvern.com, www.retsch-technology.com, ou encore www.occhio.be.

Au moyen de l'appareillage Alpaga 500 Nano, on réalise pour chaque échantillon testé des acquisitions sur 10000 particules et on calcule les paramètres d'allongement et d'émoussé pour chaque particule.

Les outils mathématiques utilisés pour leur calcul sont développés dans la thèse de doctorat de E. Pirard (1993, Université de Liège, 253 p.) intitulée « Morphométrie euclidienne des figures planes. Applications à l'analyse des matériaux granulaires ». Le document, intitulé « The descriptive and quantitative representation of particle shape and morphology » est disponible sous la référence ISO/DIS 9276-6.

La sphéricité est évaluée à l'aide des deux paramètres suivants, comme par exemple décrit dans la demande WO2008152319:
- l'émoussé (« roundness » en langue anglaise) est exprimé en pourcentages, et est calculé à partir des moments de la distribution des cercles inscrits dans la particule tangents aux points du contour de la particule, selon un filtrage complexe ; il est représentatif de la variation du rayon de courbure des particules et traduit la maturité d'un grain dans un processus d'abrasion. Les aspérités douces sont plus significatives que les aspérités très saillantes. Plus la forme des particules se rapproche de la sphéricité parfaite, plus l'émoussé est proche de 100% ;
- l'allongement, exprimé en pourcentage, est parfois appelé dans la littérature « facteur de forme elliptique » et se calcule à partir du rapport entre le grand et le petit axe de l'ellipse inertielle de la particule, a et b, selon la formule A = 100 (1-a/b). L'allongement est égal à 0 pour une particule sphérique, il croît pour des formes s'écartant de la sphéricité et tend vers 100 pour une particule allongée telle qu'une fibre.

### Résistance mécanique des adsorbants zéolithiques

La résistance à l'écrasement d'un lit d'adsorbants zéolithiques tels que décrits dans la présente invention est caractérisée selon la méthode Shell série SMS1471-74 (Shell Method Series SMS1471-74 « Détermination of Bulk Crushing Strength of Catalysts. Compression-Sieve Method »), associée à l'appareil « BCS Tester » commercialisé par la société Vinci Technologies. Cette méthode, initialement destinée à la caractérisation de catalyseurs de 3 mm à 6 mm est basée sur l'utilisation d'un tamis de 425 µm qui va permettre notamment de séparer les fines créées lors de l'écrasement. L'utilisation d'un tamis de 425 µm reste adaptée pour des adsorbants zéolithiques de diamètre supérieur à 1,6 mm, mais doit être adaptée selon la granulométrie des adsorbants que l'on cherche à caractériser.

Les adsorbants utilisées dans le procédé selon la présente invention, de préférence sous forme de billes, ont un diamètre moyen en volume, compris entre 0,2 mm et 0,6 mm, et en particulier comprise entre 0,3 mm et 0,6 mm. Par conséquent, un tamis de 100 µm est utilisé à la place du tamis de 425 µm mentionné dans la méthode Shell standard SMS1471-74.

Le protocole de mesure est le suivant : un échantillon de 20 cm³ d'adsorbants zéolithiques, préalablement tamisé avec le tamis adapté (100 µm) et préalablement séché à l'étuve pendant au moins 2 heures à 250°C (au lieu de 300°C mentionné dans la méthode Shell standard SMS1471-74), est placé dans un cylindre métallique de section interne connue. Une force croissante est imposée par paliers sur cet échantillon par l'intermédiaire d'un piston, à travers un lit de 5 cm³ de billes d'acier afin de mieux répartir la force exercée par le piston sur les agglomérés d'adsorbants (utilisation de billes de 2 mm de diamètre pour des particules de forme sphérique de diamètre strictement inférieur à 1,6 mm). Les fines obtenues aux différents paliers de pression sont séparées par tamisage (tamis adapté de 100 µm) et pesées.

La résistance à l'écrasement en lit est déterminée par la pression en mégaPascal (MPa) pour laquelle la quantité de fines cumulées passant à travers le tamis s'élève à 0,5% pondéral de l'échantillon. Cette valeur est obtenue en traçant sur un graphique la masse de fines obtenue en fonction de la force appliquée sur le lit d'adsorbant et en interpolant à 0,5 % massique de fines cumulées. La résistance mécanique à l'écrasement en lit est typiquement comprise entre quelques centaines de kPa et quelques dizaines de MPa et généralement comprise entre 0,3 MPa et 3,2 MPa. La précision est de manière classique inférieure à 0,1 MPa.

### Volume microporeux, surface externe et diamètre des mésopores

La cristallinité des adsorbants est également évaluée par mesure de leur volume microporeux en le comparant à celui d'une référence appropriée (adsorbant à taux de liant identique, avec zéolithe 100% cristalline dans des conditions de traitements cationiques identiques ou zéolithe théorique). Ce volume microporeux est déterminé à partir de la mesure de l'isotherme d'adsorption de gaz, tel que l'azote, à sa température de liquéfaction.

Préalablement à l'adsorption, l'adsorbant zéolithique est dégazé entre 300°C et 450°C pendant une durée comprise entre 9 heures et 16 heures, sous vide (P < 6,7.10⁻⁴ Pa). La mesure de l'isotherme d'adsorption d'azote à 77K est ensuite effectuée sur un appareil de type ASAP 2020 M de Micromeritics, en prenant au moins 35 points de mesure à des pressions relatives de rapport P/P₀ compris entre 0,002 et 1.

Le volume microporeux ainsi que la surface externe sont déterminés à partir de l'isotherme obtenue, par la méthode du t-plot en appliquant la norme ISO 15901-3:2007 et en calculant l'épaisseur statistique t par l'équation de Harkins-Jura. Le volume microporeux et la surface externe sont obtenus par régression linéaire sur les points du t-plot compris entre 0,45 et 0,57 nm, respectivement à partir de l'ordonnée à l'origine et de la pente de la régression linéaire. Le volume microporeux évalué s'exprime en cm³ d'adsorbat liquide par gramme d'adsorbant anhydre. La surface externe s'exprime en m² par gramme d'adsorbant anhydre.

L'interprétation de l'isotherme d'adsorption d'azote à 77K par la méthode de Barrett-Joyner-Halenda (méthode BJH, proposée en 1951) permet en outre d'obtenir la distribution de taille de pores, et notamment la distribution des mésopores. La distribution en volume de taille de mésopores est représentée par la courbe dV/d(d) en fonction du diamètre moyen des pores.

### Volume macroporeux et mésoporeux et densité de grain

Les volumes macroporeux et mésoporeux sont mesurés par porosimétrie par intrusion de mercure. Un porosimètre à mercure type Autopore^{®} 9500 de Micromeritics est utilisé pour analyser la répartition du volume poreux contenu dans les macropores et dans les mésopores.

La méthode expérimentale, décrite dans le manuel opératoire de l'appareil faisant référence à la norme ASTM D4284-83, consiste à placer un échantillon d'adsorbant (matériau granulaire zéolithique à mesurer) (de perte au feu connue) préalablement pesé, dans une cellule du porosimètre, puis, après un dégazage préalable (pression d'évacuation de 30 µm Hg pendant au moins 10 min), à remplir la cellule avec du mercure à une pression donnée (0,0036 MPa), et ensuite à appliquer une pression croissante par palier jusqu'à 400 MPa afin de faire pénétrer progressivement le mercure dans le réseau poreux de l'échantillon.

La relation entre la pression appliquée et le diamètre apparent des pores est établie en supposant des pores cylindriques, un angle de contact entre le mercure et la paroi des pores de 140° et une tension de surface du mercure de 485 dynes/cm. La quantité cumulée de mercure introduite en fonction de la pression appliquée est enregistrée. On fixe à 0,2 MPa la valeur à partir de laquelle le mercure remplit tous les vides inter-granulaires, et on considère qu'au delà le mercure pénètre dans les pores du matériau granulaire. Le volume de grain (Vg) est alors calculé en soustrayant le volume cumulé de mercure à cette pression (0,2 MPa) au volume de la cellule du porosimètre, et en divisant cette différence par la masse du matériau granulaire équivalent anhydre, c'est-à-dire la masse dudit matériau corrigée de la perte au feu. La densité de grain est l'inverse du volume de grain (Vg), et s'exprime en gramme d'adsorbant anhydre par cm³.

Le volume macroporeux du matériau granulaire est défini comme étant le volume cumulé de mercure introduit à une pression comprise entre 0,2 MPa et 30 MPa, correspondant au volume contenu dans les pores de diamètre apparent supérieur à 50 nm. Le volume mésoporeux du matériau granulaire est défini comme étant le volume cumulé de mercure introduit à une pression comprise entre 30 MPa et 400 MPa.

Dans le présent document, les volumes macroporeux et mésoporeux des adsorbants zéolithiques, exprimés en cm³.g⁻¹, sont ainsi mesurés par intrusion de mercure et rapportés à la masse de l'échantillon en équivalent anhydre, c'est-à-dire la masse dudit matériau corrigée de la perte au feu.

### Perte au feu des adsorbants zéolithiques

La perte au feu est déterminée en atmosphère oxydante, par calcination de l'échantillon à l'air à une température de 950°C ± 25°C, comme décrit dans la norme NF EN 196-2 (avril 2006). L'écart type de mesure est inférieur à 0,1%.

### Exemple A : Préparation de pâte à partir de cristaux de XPH

On prépare un mélange homogène constitué de 1600 g équivalent anhydre de cristaux de zéolithe X mésoporeuse synthétisée d'après le mode opératoire de Inayat et coll. (Angew. Chem. Int. Ed., 51, (2012), 1962-1965), de 350 g équivalent anhydre de kaolin, de 130 g de silice colloïdale vendue sous la dénomination commerciale de Klebosol^{®} 30 (contenant 30% en poids de SiO₂ et 0,5% de Na₂O) ainsi que de la quantité d'eau qui permet l'extrusion du mélange. La perte au feu de la pâte avant mise en forme est de 44%.

### Exemple B : Préparation de pâte à partir de cristaux de X de diamètre 1,6 µm

On prépare un mélange homogène constitué de 1600 g équivalent anhydre de cristaux de zéolithe X synthétisée selon le mode opératoire décrit dans le mode opératoire B de la demande de brevet WO2008/009845), de 300 g équivalent anhydre de kaolin, de 130 g de silice colloïdale vendue sous la dénomination commerciale de Klebosol^{®} 30 (contenant 30% en poids de SiO₂ et 0,5% de Na₂O) ainsi que de la quantité d'eau qui permet l'extrusion du mélange.

### Exemple C : Préparation d'adsorbants agglomérés par extrusion / concassage / tamisage

À partir de la pâte préparée selon le mode opératoire A ou B ci-dessus, on forme des extrudés de 1,6 mm de diamètre. Les extrudés sont séchés une nuit en étuve ventilée à 80°C. Ils sont ensuite calcinés pendant 2 heures à 550°C sous balayage à l'azote, puis 2 heures à 550°C sous balayage à l'air sec décarbonaté puis concassés de manière à récupérer des grains par tamisage selon la granulométrie souhaitée, à savoir : des concassés de granulométrie 0,4 mm (obtenus par sélection des concassés de diamètre équivalent compris entre 0,3 et 0,5 mm de sorte que le diamètre moyen en volume est égal à 0,4 mm).

### Exemple D : Préparation d'adsorbants agglomérés par granulation / tamisage

On utilise la pâte préparée selon le mode opératoire A ou B sur une assiette granulatrice afin de réaliser des billes d'adsorbant aggloméré. Une sélection par tamisage des billes obtenues est réalisée selon la granulométrie souhaitée, à savoir :
- Billes de granulométrie 0,4mm : sélection des billes de diamètre compris entre 0,3 et 0,5 mm de sorte que le diamètre moyen en volume est égal à 0,4 mm ;
- Billes de granulométrie 0,54mm : sélection des billes de diamètre compris entre 0,3 et 0,8 mm de sorte que le diamètre moyen volume est égal à 0,54 mm ;
- Billes de granulométrie 0,7mm : sélection des billes de diamètre compris entre 0,4 et 1,0 mm de sorte que le diamètre moyen en volume est égal à 0,7 mm.

Les billes sont séchées une nuit en étuve ventilée à 80°C. Elles sont ensuite calcinées pendant 2 heures à 550°C sous balayage à l'azote, puis 2 heures à 550°C sous balayage à l'air sec décarbonaté.

### Exemple E : Zéolithisation (optionnelle)

Les granulés ou les billes obtenus aux exemples C ou D sont placés dans un réacteur en verre muni d'une double enveloppe régulée à une température de 100°C ± 1°C puis on ajoute 1,5 L d'une solution aqueuse d'hydroxyde de sodium de concentration 100 g.L⁻¹ et on laisse le milieu réactionnel sous agitation pendant 3 heures. On procède ensuite au lavage des granulés en 3 opérations successives de lavage à l'eau suivi de la vidange du réacteur. On s'assure de l'efficacité du lavage en mesurant le pH final des eaux de lavage qui est compris entre 10 et 10,5.

### Exemple F : Échange - activation

Les granulés ou les billes obtenus à l'étape C ou D ou éventuellement E sont échangés au moyen d'une solution aqueuse de chlorure de baryum (BaCl₂) 0,7 M à 95°C en 4 étapes. À chaque étape, le ratio volume de solution / masse de solide est de 20 mL.g⁻¹ et l'échange est poursuivi pendant 4 heures à chaque fois. Entre chaque échange, le solide est lavé plusieurs fois de manière à le débarrasser des excédents de BaCl₂. Il est ensuite séché à 80°C pendant 2 heures et enfin activé à une température de 250°C pendant 2 heures sous courant d'azote.

### Exemple 1 : (exemple comparatif)

On prépare un adsorbant sous forme de concassés (i.e. éléments non sphériques) de granulométrie 0,4 mm à partir des étapes successives décrites aux exemples A, C et E. Cet adsorbant est caractérisé selon les techniques décrites.

Le taux d'échange global en baryum est de 97% et la perte au feu (mesurée à 900°C) est de 5,5%. Le volume microporeux et la surface externe mesurés selon la méthode du t-plot à partir de l'isotherme d'adsorption d'azote à 77K après dégazage sous vide à 400°C pendant 10 heures sont respectivement 0,192 cm³.g⁻¹ et 70 m².g⁻¹.

Le volume total contenu dans les macropores et les mésopores (somme du volume macroporeux et du volume mésoporeux) mesuré par intrusion de mercure, est de 0,33 cm³.g⁻¹. Le ratio (volume macroporeux)/(volume macroporeux + volume mésoporeux) est égal à 0,6. La densité de grain de l'adsorbant (calculée en divisant la masse de l'échantillon équivalent anhydre par le volume de l'échantillon évalué d'après le volume de mercure introduit à une pression de 0,2MPa) est de 1,10 g.cm⁻³ équivalent anhydre.

L'adsorbant est utilisé pour remplir une colonne en inox de 0,77cm de diamètre interne et 1 m de long, équipée à l'extrémité d'une grille filtrante. Au minimum trois remplissages sont réalisés avec l'adsorbant dont la perte au feu était mesurée à 5,5% afin d'estimer une valeur moyenne de densité de remplissage de l'adsorbant : on obtient 0,730 ± 0,010 g.cm⁻³, ce qui permet d'évaluer à 37,3 ± 0,8%, la porosité de lit, c'est-à-dire la proportion de vide entre les grains d'adsorbant par rapport au volume de colonne. L'émoussé de l'adsorbant sous forme de concassé est de 57%, et l'allongement est de 9%.

### Exemple 2 : (selon l'invention)

On prépare un adsorbant sous forme de billes de granulométrie 0,4mm à partir des étapes successives décrites aux exemples A, D et E. L'adsorbant ainsi préparé est caractérisé selon les techniques décrites.

Le taux d'échange global en baryum est de 96,4% et la perte au feu (mesurée à 900°C) est de 5,40%. Le volume microporeux et la surface externe mesurés selon la méthode du t-plot à partir de l'isotherme d'adsorption d'azote à 77K après dégazage sous vide à 400°C pendant 10 heures sont respectivement 0,195 cm³.g⁻¹ et 63 m².g⁻¹.

Le volume total contenu dans les macropores et les mésopores (somme du volume macroporeux et du volume mésoporeux) mesuré par intrusion de mercure, est de 0,31 cm³.g⁻¹. Le ratio (volume macroporeux)/(volume macroporeux + volume mésoporeux) est égal à 0,65. La densité de grain de l'adsorbant (calculée en divisant la masse de l'échantillon équivalent anhydre par le volume de l'échantillon évalué d'après le volume de mercure introduit à une pression de 0,2 MPa) est de 1,14 g.cm⁻³ équivalent anhydre.

L'adsorbant ainsi préparé est utilisé pour remplir une colonne en inox de 0,77 cm de diamètre interne et 1 m de long, équipée à l'extrémité d'une grille filtrante. Plusieurs remplissages avec l'adsorbant dont la perte au feu était mesurée à 5,4% sont réalisés afin d'estimer une valeur moyenne de densité de remplissage de l'adsorbant : on obtient 0,764 ± 0,008 g.cm⁻³, ce qui permet d'évaluer la porosité de lit, c'est-à-dire la proportion de vide entre les grains d'adsorbant par rapport au volume de colonne, à 33,0 ± 0,7%.
L'émoussé de l'adsorbant sous forme de billes est de 77%, et l'allongement est de 3%.

La comparaison des adsorbants de l'exemple 1 et de l'exemple 2, montre que la porosité de lit lors d'un remplissage avec des agglomérés ayant un émoussé de 77%, indiquant une morphologie proche de la sphéricité, est inférieur de plus de 4% absolu par rapport à la porosité de lit obtenue lors d'un remplissage avec des agglomérés concassés ayant un émoussé de 57%. Un lit ayant une porosité de lit plus faible indique une plus grande compacité des agglomérés, c'est-à-dire une plus grande quantité d'adsorbant par volume de lit, ce qui aura pour conséquence une plus grande productivité lors de l'utilisation de ces adsorbants en séparation du para-xylène. En effet, la productivité correspond à la quantité de para-xylène produit par unité de temps et par volume de lit.

### Exemple 3 : Tests de remplissage et de performance de séparation de xylènes

On prépare des adsorbants sous forme de billes d'émoussé égal à 80%, de différentes granulométries : 0,4 mm - 0,54 mm (selon l'invention) et 0,7 mm (comparatif) à partir des étapes successives suivantes :
- étapes A, D et F pour les adsorbants à partir de cristaux de zéolithe FAU de type X à porosité hiérarchisée (zéolithe FAU X mésoporeuse)
- étapes B, D, E et F pour les adsorbants à partir de cristaux de X de diamètre 1,6 µm.

On teste ces différents adsorbants pour évaluer leur performance en séparation du para-xylène sur une unité pilote de chromatographie à contre-courant simulé constituée de 12 colonnes en série de 2 cm de diamètre. Plusieurs longueurs de colonne peuvent être utilisées : 0,5 m, 1 m ou 2 m. La circulation entre la dernière et la première colonne se fait au moyen d'une pompe de recyclage. À chaque liaison intercolonne, on peut injecter soit une charge à séparer soit du désorbant. On peut également soutirer soit un raffinat soit un extrait.

L'ensemble des colonnes et du vannage de distribution est placé dans une étuve à 175°C, et la pression est maintenue au-dessus de 15 bars (1,5 MPa). Les décalages des différents points d'injection ou de soutirage sont simultanés selon un temps de permutation qui peut être ajusté. Les lits sont répartis en 4 zones chromatographiques selon la configuration suivante :
- 2 lits entre l'injection de désorbant et le soutirage d'extrait
- 5 lits entre le soutirage d'extrait et l'injection de charge
- 3 lits entre l'injection de charge et le soutirage de raffinat
- 2 lits entre le soutirage de raffinat et l'injection de désorbant.

La charge est composée de 21,3% de para-xylène, de 19,6% d*'ortho*-xylène, de 45,1% de *méta*-xylène et de 14,0% d'éthylbenzène. Le désorbant utilisé est le para-diéthylbenzène. Les pourcentages ci-dessus sont exprimés en pourcentages en poids.

Dans un premier temps, on réalise un test à partir d'un adsorbant selon l'art antérieur, noté « A(comp) » dans la suite. A(comp) est un adsorbant de granulométrie 0,7 mm préparé à partir de cristaux de zéolithe X de 1,6 µm de diamètre moyen en nombre, préparé à l'identique de l'exemple 4 du brevet WO2008/009845, sauf pour l'étape de mise en forme, réalisée par granulation afin d'obtenir des billes sphériques (étape D) et non des concassés, ce qui permet d'optimiser le remplissage de la colonne via une porosité de lit plus réduite grâce à la sphéricité des agglomérés (émoussé de 80%). Ce test permet de déterminer les débits d'injection de charge et de désorbant nécessaires pour obtenir du para-xylène avec une pureté de 99,7% et un rendement d'au moins 98%.

En utilisant les colonnes de longueur 2 m remplies avec l'adsorbant de référence, on obtient le para-xylène à l'extrait avec un rendement de 98,5% en injectant la charge à un débit de 40,8 g.min⁻¹ et le désorbant à un débit de 48,7 g.min⁻¹. La différence de pression mesurée entre la première et la dernière colonne est de 3,5 bar (0,35 MPa).

Par la suite, l'ensemble des adsorbants sont testés en appliquant les mêmes débits d'injection, c'est-à-dire les débits de charge et de désorbant, et en réglant les débits de zone au sein de l'adsorbeur de façon à obtenir une pureté 99,7%. Les rendements obtenus sont reportés dans le Tableau 1, sachant que le niveau minimum de rendement requis est de 98%.

On remplit tout d'abord les colonnes de longueur 2 m avec des billes de même granulométrie que l'adsorbant de référence à savoir 0,7 mm, d'un adsorbant préparé avec des cristaux XPH. En appliquant les débits d'injection de charge et de désorbant, on atteint un rendement de 98,5%. Dans ces conditions d'opération, la différence de pression (ΔP) mesurée entre la première et la dernière colonne augmente par rapport au cas de référence, et passe à 3,7 bar (0,37 MPa). Comptetenu de cette augmentation de pression, l'homme du métier ne serait pas enclin à diminuer le diamètre de billes d'adsorbant préparé à partir de cristaux XPH, étant donné que la perte de charge linéaire augmente avec la diminution de la granulométrie des billes.

Pourtant, les tests utilisant des billes de granulométrie réduite d'adsorbant à base de cristaux de zéolithe X à porosité hiérarchisée (XPH), c'est-à-dire un adsorbant selon l'invention (noté « A(inv) » dans la suite de la présente description), montrent qu'il est possible d'obtenir du para-xylène (pureté de 99,7%) avec un rendement d'au moins 98%, en injectant les débits de charge et de désorbant identiques au cas de référence, respectivement de 40,8 g.min⁻¹ et 48,7 g.min⁻¹, tout en utilisant des colonnes plus courtes, c'est-à-dire en utilisant une quantité d'adsorbant réduite, et ceci, sans augmentation de perte de charge au sein de l'unité. Les tests comparatifs réalisés avec les mêmes longueurs de colonne et des billes de même granulométrie d'adsorbant préparé avec des cristaux de zéolithe X conventionnelle de diamètre 1,6 µm ne permettent pas d'obtenir le para-xylène aux performances requises (rendement inférieur à 98%).

Par exemple, avec un adsorbant selon l'invention de granulométrie 0,54 mm, on peut utiliser des colonnes de 1 m et produire le para-xylène avec un rendement de 98,5% ou avec un adsorbant selon l'invention de granulométrie 0,4 mm, on peut utiliser des colonnes de 0,5 m et produire le para-xylène avec un rendement de 98,7%. Pour ces tests, la différence de pression mesurée entre la première et la dernière colonne est de 3,4 bar (0,34 MPa) et 3,5 bar (0,35 MPa) respectivement, ce qui est inférieur ou identique au cas de référence.

Dans cet exemple il apparait que l'utilisation d'adsorbants zéolithiques selon l'invention, c'est-à-dire les deux adsorbants à base de cristaux de type XPH et de granulométrie (diamètre moyen en volume) 0,4 mm et 0,54 mm permettent de réduire la longueur de colonne pour un même débit de charge injecté et de performances pureté/rendement ce qui permet de d'augmenter la productivité par rapport aux adsorbants conventionnels (à base de cristaux de X de type conventionnels) ; en effet, la productivité correspond à la quantité de para-xylène produit par unité de temps et par volume de lit.

**-- Tableau 1 --**

| ***Adsorbant*** | A(comp) | A(comp) | A(comp) | A(comp) | A(inv) | A(inv) |
|---|---|---|---|---|---|---|
| ***Type de cristaux*** | X | X | X | XPH | XPH | XPH |
| ***diamètre moyen en volume de l'adsorbant (mm)*** | 0,4 | 0,54 | 0,7 | 0,7 | 0,4 | 0,54 |
| ***Longueur colonne (m)*** | 0,5 | 1 | 2 | 2 | 0,5 | 1 |
| ***Rendement*** | 64,6% | 93,9% | 98,5% | 98,4% | 98,7% | 98,5% |
| ***Vitesse maximale (cm.sec⁻¹)*** | 1,6 | 1,5 | 1,3 | 1,4 | 1,8 | 1,5 |
| ***ΔP (MPa)*** | 0,31 | 0,34 | 0,35 | 0,37 | 0,35 | 0,34 |

## Revendications

1. Adsorbant zéolithique sous forme de billes pour lequel :
- la teneur en phase cristalline sous forme de zéolithe FAU de type X à porosité hiérarchisée, c'est-à-dire possédant à la fois des micropores et des mésopores identifiables par observation au moyen d'un Microscope Électronique à Transmission, est comprise entre 50% et 99% en poids, de préférence de 80% à 98% en poids, par rapport à la masse totale de l'adsorbant, la teneur en phase cristalline étant mesurée par analyse par diffraction de rayons X ;
- la teneur en ions baryum (Ba²⁺), exprimée en oxyde de baryum (BaO) est supérieure à 10%, de préférence supérieure à 15%, de manière très préférée supérieure à 20%, de manière encore plus préférée supérieure à 23%, voire supérieure à 33% en poids par rapport à la masse totale de l'adsorbant, avantageusement, la teneur en oxyde de baryum est comprise entre 23% et 42%, et typiquement entre 30% et 40%, en poids par rapport au poids total de l'adsorbant, la teneur en ions baryum étant mesurée par analyse chimique par fluorescence de rayons X selon la norme NF EN ISO 12677 : 2011 ;
- la teneur en ions potassium (K⁺), exprimée en oxyde de potassium (K₂O) est inférieure à 25%, de préférence comprise entre 0% et 20%, de manière encore plus préférée comprise entre 0% et 15% et de manière très préférée de 0% à 10% en poids par rapport à la masse totale de l'adsorbant, la teneur en ions potassium étant mesurée par analyse chimique par fluorescence de rayons X selon la norme NF EN ISO 12677 : 2011;
- le diamètre moyen en volume est compris entre 0,2 mm et 0,6 mm, et de préférence compris entre 0,3 mm et 0,6 mm, mesuré par analyse de la distribution granulométrique d'un échantillon d'adsorbant par imagerie selon la norme ISO 13322-2:2006 et calculé à partir de la distribution granulométrique en appliquant la norme ISO 9276-2:2001 ; et
dont les caractéristiques de sphéricité sont telles que :
- l'émoussé moyen est compris entre 70% et 100%, de préférence entre 80% et 100%, de manière préférée entre 85 % et 100%, de manière encore plus préférée entre 90 % et 100%, mesuré par la microscopie électronique à balayage;
- le pourcentage de particules ayant un allongement de 0% est compris entre 5 et 100, de préférence entre 10 et 100 et de manière préférée entre 25 et 100, mesuré par la microscopie électronique à balayage ; et
- la surface externe, mesurée par adsorption d'azote et calculée par la méthode du t-plot à partir de l'isotherme d'adsorption d'azote à une température de 77K, après dégazage sous vide (P < 6,7.1 0 4 Pa), à une température comprise entre 300°C et 450°C pendant une durée allant de 9 heures à 16 heures, en appliquant la norme ISO 15901-3:2007, est supérieure à 20 m².g⁻¹.

2. Adsorbant zéolithique selon la revendication 1, pour lequel la surface externe, mesurée par adsorption d'azote, est supérieure à 30 m².g⁻¹, et de préférence encore comprise entre 30 m².g⁻¹ et 200 m².g⁻¹, et plus préférentiellement comprise entre 30 m².g⁻¹ et 150 m².g⁻¹.

3. Adsorbant zéolithique selon la revendication 1 ou la revendication 2, pour lequel la surface externe, mesurée par adsorption d'azote, est supérieure à 20 m².g⁻¹, de préférence supérieure à 30 m².g⁻¹, et de préférence encore comprise entre 30 m².g⁻¹ et 200 m².g⁻¹, et plus préférentiellement comprise entre 30 m².g⁻¹ et 150 m².g⁻¹, et ladite surface externe est associée à une population de mésopores de diamètre moyen compris entre 2 nm et 50 nm, ce diamètre étant déterminé par la méthode de Barrett-Joyner-Halenda à partir de la branche d'adsorption de l'isotherme de physisorption d'azote à 77K.

4. Adsorbant zéolithique selon l'une quelconque des revendications précédentes, pour lequel la zéolithe FAU de type X à porosité hiérarchisée présente une surface externe, définie par la méthode du t-plot à partir de l'isotherme d'adsorption d'azote à une température de 77K, après dégazage sous vide (P < 6,7.10⁻⁴ Pa), à une température comprise entre 300°C et 450°C pendant une durée allant de 9 heures à 16 heures, comprise entre 40 m².g⁻¹ et 400 m².g⁻¹, de préférence entre 40 m².g⁻¹ et 200 m².g⁻¹, de préférence encore entre 40 m².g⁻¹ et 150 m².g⁻¹.

5. Adsorbant zéolithique selon l'une quelconque des revendications précédentes, qui comprend un liant, ledit liant comprenant une argile ou un mélange d'argiles choisies parmi les kaolins, kaolinites, nacrites, dickites, halloysites, attapulgites, sépiolites, montmorillonites, bentonites, illites et métakaolins, ainsi que les mélanges de deux ou plusieurs d'entre elles en toutes proportions.

6. Adsorbant zéolithique selon l'une quelconque des revendications précédentes, dont le pourcentage de particules ayant un allongement inférieur à 10% est compris entre 50 et 100 et de préférence entre 70 et 100 et de manière préférée entre 80 et 100.

7. Adsorbant zéolithique selon l'une quelconque des revendications précédentes, présentant une densité de grain comprise entre 1,0 g.cm⁻³ et 1,4 g.cm⁻³, et de manière préférée entre 1,1 g.cm⁻³ et 1,3 g.cm⁻³ telle que mesurée par intrusion de mercure selon la norme ASTM D4284-83 (exprimée par rapport à la masse sèche de l'adsorbant zéolithique) et un volume total contenu dans les macropores et les mésopores (somme du volume macroporeux et du volume mésoporeux) mesuré par intrusion de mercure selon la norme ASTM D4284-83, compris entre 0,20 cm³.g⁻¹ et 0,40 cm³.g⁻¹ et de préférence compris entre 0,20 cm³.g⁻¹ et 0,35 cm³.g⁻¹ (exprimé par rapport à la masse équivalent anhydre de l'adsorbant zéolithique).

8. Adsorbant zéolithique selon l'une quelconque des revendications précédentes, pour lequel le ratio (volume macroporeux)/(volume macroporeux + volume mésoporeux) est compris entre 0,2 et 1, de manière préférée est compris entre 0,4 et 0,8, et de manière encore plus préférée est compris entre 0,45 et 0,65, le volume macroporeux et le volume mésoporeux étant mesuré par intrusion de mercure selon la norme ASTM D4284-83.

9. Utilisation d'au moins un adsorbant zéolithique selon l'une quelconque des revendications 1 à 8, pour la séparation de para-xylène à partir de coupes aromatiques à 8 atomes de carbone.

10. Procédé de séparation de para-xylène à partir de coupes d'isomères C₈ aromatiques **caractérisé en ce qu'**il comprend une étape d'adsorption, en phase gaz ou en phase liquide, consistant à utiliser un adsorbant zéolithique tel que défini dans l'une quelconque des revendications 1 à 8.

11. Procédé de séparation des xylènes selon la revendication 10, **caractérisé en ce qu'**il comprenant au moins les étapes suivantes :
a) mise en contact, dans des conditions d'adsorption adéquates, de la charge avec un lit d'adsorbant zéolithique, de manière à adsorber préférentiellement le para xylène,
b) mise en contact, dans des conditions de désorption, du lit d'adsorbant avec un désorbant,
c) soutirage du lit d'adsorbant d'un flux contenant le désorbant et les produits de la charge des moins sélectivement adsorbés,
d) soutirage du lit d'adsorbant d'un flux contenant le désorbant et le para-xylène,
e) séparation du flux issu de l'étape c) en un premier flux contenant le désorbant et un second flux contenant les produits de la charge les moins sélectivement adsorbés, et
f) une séparation d'un flux issu de l'étape d) en un premier flux contenant le désorbant et un second flux du para-xylène à un niveau de pureté supérieure ou égale à 75%.

12. Procédé de séparation du paraxylène selon l'une des revendications 10 à 11, dans lequel le désorbant est choisi parmi le toluène et le para-diéthylbenzène.

## Patentansprüche

1. Zeolithisches Adsorbens in Kugelform, wobei:
- der Gehalt in kristalliner Phase in Form von FAU-Zeolith vom Typ X mit hierarchisierter Porosität, das heißt, mit sowohl Mikroporen als auch Makroporen, die durch Beobachtung mittels eines Transmissions-Elektronenmikroskops identifizierbar sind, zwischen 50 und 99 Gew.-%, vorzugsweise 80 bis 98 Gew.-%, im Verhältnis zur Gesamtmasse des Adsorbens liegt, wobei der Gehalt in kristalliner Phase durch Röntgenstrahldiffraktionsanalyse gemessen wird;
- der Gehalt an Bariumionen (Ba²⁺), ausgedrückt in Bariumoxid (BaO), über 10 Gew.-%, vorzugsweise über 15 Gew.-%, sehr bevorzugt über 20 Gew.-%, noch bevorzugter über 23 Gew.-%, ja sogar über 33 Gew.-% im Verhältnis zur Gesamtmasse des Adsorbens ist, wobei in vorteilhafter Weise der Gehalt an Bariumoxid zwischen 23 und 42 Gew.-% und in typischer Weise zwischen 30 und 40 Gew.-% im Verhältnis zum Gesamtgewicht des Adsorbens liegt, wobei der Gehalt an Bariumionen durch chemische Röntgenstrahlfluoreszenzanalyse gemäß der Norm NF EN ISO 12677:2011 gemessen wird;
- der Gehalt an Kaliumionen (K⁺), ausgedrückt in Kaliumoxid (K₂O), unter 25 Gew.-%, vorzugsweise zwischen 0 und 20 Gew.-%, noch bevorzugter zwischen 0 und 15 Gew.-% und sehr bevorzugt von 0 bis 10 Gew.-% im Verhältnis zur Gesamtmasse des Adsorbens liegt, wobei der Gehalt an Kaliumionen durch chemische Röntgenstrahlfluoreszenzanalyse gemäß der Norm NF EN ISO 12677:2011 gemessen wird;
- der mittlere Volumendurchmesser zwischen 0,2 mm und 0,6 mm und vorzugsweise zwischen 0,3 mm und 0,6 mm, gemessen durch Analyse der granulometrischen Verteilung in einer Probe des Adsorbens durch bildliche Darstellung gemäß der Norm ISO 13322-2:2006 und berechnet auf der Basis der granulometrischen Verteilung bei Anwendung der Norm ISO 9276-2:2001, liegt; und
dessen Sphärizitätsmerkmale derart sind, dass:
- die mittlere Stumpfheit zwischen 70 und 100%, vorzugsweise zwischen 80 und 100%, bevorzugt zwischen 85 und 100 %, noch bevorzugter zwischen 90 und 100 %, gemessen durch Rasterelektronenmikroskopie, liegt;
- der Prozentsatz der Partikel mit einer Längung von 0 % zwischen 5 und 100, vorzugsweise zwischen 10 und 100 und bevorzugt zwischen 25 und 100, gemessen durch Rasterelektronenmikroskopie, liegt; und
- die externe Oberfläche, gemessen durch Stickstoffadsorption und berechnet durch die t-plot-Methode auf der Basis der Stickstoffadsorptionsisotherme bei einer Temperatur von 77 K nach Entgasung im Vakuum (P < 6,7.10⁻⁴ Pa), bei einer Temperatur zwischen 300 °C und 450 °C während einer Dauer von 9 Stunden bis 16 Stunden, bei Anwendung der Norm ISO 15901-3:2007, größer als 20 m².g⁻¹ ist.

2. Zeolithisches Adsorbens nach Anspruch 1, wobei die externe Oberfläche, gemessen durch Stickstoffadsorption, größer als 30 m².g⁻¹ ist und vorzugsweise ebenfalls zwischen 30 m².g⁻¹ und 200 m².g⁻¹ und noch vorzugsweiser zwischen 30 m².g⁻¹ und 150 m².g⁻¹ liegt.

3. Zeolithisches Adsorbens nach Anspruch 1 oder Anspruch 2, wobei die externe Oberfläche, gemessen durch Stickstoffadsorption, größer als 20 m².g⁻¹, vorzugsweise größer als 30 m².g⁻¹ ist und vorzugsweise ebenfalls zwischen 30 m².g⁻¹ und 200 m².g⁻¹ und noch vorzugsweiser zwischen 30 m².g⁻¹ und 150 m².g⁻¹ liegt und die externe Oberfläche einer Mesoporenpopulation mit einem mittleren Durchmesser zugeordnet ist, der zwischen 2 nm und 50 nm liegt, wobei dieser Durchmesser durch die Barrett-Joyner-Halenda-Methode auf der Basis des Adsorptionsasts der Stickstoffphysisorptionsisotherme bei 77 K bestimmt wird.

4. Zeolithisches Adsorbens nach einem der vorangehenden Ansprüche, wobei der FAU-Zeolith vom Typ X mit hierarchisierter Porosität eine externe Oberfläche, definiert durch die t-plot-Methode auf der Basis der Stickstoffadsorptionsisotherme bei einer Temperatur von 77 K nach Entgasung im Vakuum (P < 6,7.10⁻⁴ Pa) bei einer Temperatur zwischen 300 °C und 450 °C während einer Dauer von 9 Stunden bis 16 Stunden zwischen 40 m².g⁻¹ und 400 m².g⁻¹, vorzugsweise zwischen 40 m².g⁻¹ und 200 m².g⁻¹, vorzugsweise ebenfalls zwischen 40 m².g⁻¹ und 150 m².g⁻¹ aufweist.

5. Zeolithisches Adsorbens nach einem der vorangehenden Ansprüche, das ein Bindemittel aufweist, wobei das Bindemittel einen Ton oder ein Tongemisch umfasst, ausgewählt aus den Kaolinen, Kaoliniten, Nacriten, Dickiten, Halloysiten, Attapulgiten, Sepioliten, Montmorilloniten, Bentoniten, Illiten und Metakaolinen sowie den Gemischen von zwei oder mehreren aus diesen in allen Verhältnissen.

6. Zeolithisches Adsorbens nach einem der vorangehenden Ansprüche, dessen Prozentsatz der Partikel, die eine Längung unter 10 % aufweisen, zwischen 50 und 100 und vorzugsweise zwischen 70 und 100 und vorzugsweise zwischen 80 und 100 liegt.

7. Zeolithisches Adsorbens nach einem der vorangehenden Ansprüche, das eine Korndichte zwischen 1,0 g.cm⁻³ und 1,4 g.cm⁻³ und vorzugsweise zwischen 1,1 g.cm⁻³ und 1,3 g.cm⁻³, so wie durch Quecksilberintrusion gemäß der Norm ASTM D4284-83 (ausgedrückt im Verhältnis zu Trockenmasse des zeolithischen Adsorbens) gemessen, und ein in den Makroporen und den Mesoporen (Summe des makroporösen Volumens und des mesoporösen Volumens) enthaltenes Gesamtvolumen, gemessen durch Quecksilberintrusion gemäß der Norm ASTM D4284-83 zwischen 0,20 cm³.g⁻¹ und 0,40 cm³.g⁻¹ und vorzugsweise zwischen 0,20 cm³.g⁻¹ und 0,35 cm³.g⁻¹ (ausgedrückt im Verhältnis zu der wasserfreien Äquivalentmasse des zeolithischen Adsorbens) aufweist.

8. Zeolithisches Adsorbens nach einem der vorangehenden Ansprüche, wobei das Verhältnis (makroporöses Volumen)/(makroporöses Volumen + mesoporöses Volumen) zwischen 0,2 und 1, vorzugsweise zwischen 0,4 und 0,8 und noch bevorzugter zwischen 0,45 und 0,65 liegt, wobei das makroporöse Volumen und das mesoporöse Volumen durch Quecksilberintrusion gemäß der Norm ASTM D4284-83 gemessen wird.

9. Verwendung von mindestens einem zeolithischen Adsorbens nach einem der Ansprüche 1 bis 8 für die Trennung von Paraxylol auf der Basis aromatischer Fraktionen mit 8 Kohlenstoffatomen.

10. Verfahren zur Trennung von Paraxylol auf der Basis von aromatischen C8-Isomerfraktionen, **dadurch gekennzeichnet, dass** es einen Adsorptionsschritt in der Gas- oder in der flüssigen Phase umfasst, der darin besteht, ein zeolithisches Adsorbens nach einem der Ansprüche 1 bis 8 zu verwenden.

11. Verfahren zur Trennung von Xylolen nach Anspruch 10, **dadurch gekennzeichnet, dass** es mindestens die folgenden Schritte umfasst:
a) Inkontaktversetzen, unter adäquaten Adsorptionsbedingungen, der Charge mit einem zeolithischen Adsorptionsbett, so dass vorzugsweise Paraxylol adsorbiert wird;
b) Inkontaktversetzen, unter Desorptionsbedingungen, des Adsorptionsbetts mit einem Desorptionsmittel,
c) Entnehmen aus dem Desorptionsbett eines Stroms, der das Desorptionsmittel und die am wenigsten selektiv adsorbierten Produkte der Charge enthält,
d) Entnehmen aus dem Desorptionsbett eines Stroms, der das Desorptionsmittel und das Paraxylol enthält,
e) Trennen des Stroms aus Schritt c) in einen ersten Strom, der das Desorptionsmittel enthält, und einen zweiten Strom, der die am wenigsten selektiv adsorbierten Produkte der Charge enthält, und
f) ein Trennen eines Stroms aus Schritt d) in einen ersten Strom, der das Desorptionsmittel enthält, und einen zweiten Strom des Paraxylols in einem Reinheitsniveau von über oder gleich 75 %.

12. Verfahren zur Trennung von Paraxylol nach einem der Ansprüche 10 bis 11, wobei das Desorptionsmittel aus dem Toluen und dem Paradiethylbenzol ausgewählt ist.

## Claims

1. A zeolite adsorbent in the form of beads wherein:
- the zeolite adsorbent has a content of crystalline phase in the form of zeolite FAU of X type with hierarchical porosity, which means containing both micropores and mesopores identifiable by observation using a transmission electron microscope, of between 50% and 99% by weight, and preferably from 80% to 98% by weight, relative to the total mass of the adsorbent, the content of crystalline phase being measured by X-ray diffraction analysis;
- the zeolite adsorbent has a content of barium ions (Ba²⁺), expressed as barium oxide (BaO), of greater than 10%, preferably greater than 15%, very preferably greater than 20%, even more preferably greater than 23%, or even greater than 33% by weight relative to the total mass of the adsorbent, advantageously, the barium oxide content is between 23% and 42% and typically between 30% and 40% by weight relative to the total weight of the adsorbent, the content of barium ions being measured by X-ray fluorescence chemical analysis as described in standard NF EN ISO 12677: 2011;
- the zeolite adsorbent has a content of potassium ions (K⁺), expressed as potassium oxide (K₂O), of less than 25%, preferably between 0% and 20%, even more preferably between 0% and 15% and very preferably from 0% to 10% by weight relative to the total mass of the adsorbent, the content of potassium ions being measured by X-ray fluorescence chemical analysis as described in standard NF EN ISO 12677: 2011;
- the zeolite adsorbent has a volume-average diameter of between 0.2 mm and 0.6 mm, and preferably between 0.3 mm and 0.6 mm, measured by analysis of the particle size distribution of a sample of adsorbent by imaging according to standard ISO 13322-2:2006 and calculated from the particle size distribution by applying standard ISO 9276-2:2001; and
- the zeolite adsorbent has sphericity characteristics such that:
- the zeolite adsorbent has a mean roundness of between 70% and 100%, preferably between 80% and 100%, preferably between 85% and 100% and even more preferably between 90% and 100%, measured by the scanning electron microscopy;
- the zeolite adsorbent has a percentage of particles with an elongation of 0% of between 5 and 100, preferably between 10 and 100 and preferably between 25 and 100, measured by the scanning electron microscopy; and
- the zeolite adsorbent has an outer surface area, measured by nitrogen adsorption and calculated via the t-plot method from the nitrogen adsorption isotherm at a temperature of 77 K, after degassing under vacuum (P<6.7×10⁻⁴ Pa) at a temperature of between 300°C and 450°C for a time ranging from 9 hours to 16 hours, by applying standard ISO 15901-3:2007, of greater than 20 m².g⁻¹.

2. The zeolite adsorbent according to claim 1, wherein the zeolite adsorbent has an outer surface area, measured by nitrogen adsorption, of greater than 30 m².g⁻¹, more preferably between 30 m².g⁻¹ and 200 m².g⁻¹ and more preferentially between 30 m².g⁻¹ and 150 m².g⁻¹.

3. The zeolite adsorbent according to claim 1 or 2, wherein the zeolite adsorbent has an outer surface area, measured by nitrogen adsorption, of greater than 20 m².g⁻¹, preferably greater than 30 m².g⁻¹, more preferably between 30 m².g⁻¹ and 200 m².g⁻¹ and more preferentially between 30 m².g⁻¹ and 150 m².g⁻¹, and said outer surface area is associated with a population of mesopores with a mean diameter of between 2 nm and 50 nm, the diameter being determined via the Barrett-Joyner-Halenda method from the absorption arm of the nitrogen physisorption isotherm at 77 K.

4. The zeolite adsorbent according to any one of the preceding claims, wherein the type X zeolite FAU with hierarchical porosity has an outer surface area, defined by the t-plot method from the nitrogen adsorption isotherm at a temperature of 77 K, after degassing under vacuum (P<6.7×10⁻⁴ Pa), at a temperature of between 300°C and 450°C for a time ranging from 9 hours to 16 hours, of between 40 m².g⁻¹ and 400 m².g⁻¹, preferably between 40 m².g⁻¹ and 200 m².g⁻¹ and more preferably between 40 m².g⁻¹ and 150 m².g⁻¹.

5. The zeolite adsorbent according to any one of the preceding claims, wherein the zeolite adsorbent comprises a binder, said, binder comprising a clay or a mixture of clays selected from the group consisting of kaolins, kaolinites, nacrites, dickites, halloysites, attapulgites, sepiolites, montmorillonites, bentonites, illites and metakaolins, and also mixtures of two or more thereof in all proportions.

6. The zeolite adsorbent according to any one of the preceding claims, wherein the percentage of particles with an elongation of less than 10% is between 50 and 100, preferably between 70 and 100 and preferably between 80 and 100.

7. The zeolite adsorbent according to any one of the preceding claims, having a grain density of between 1.0 g.cm⁻³ and 1.4 g.cm⁻³ and preferably between 1.1 g.cm⁻³ and 1.3 g.cm⁻³ as measured by mercury intrusion according to standard ASTM D4284-83 (expressed relative to the dry mass of the zeolite adsorbent) and a total volume contained in the macropores and the mesopores (sum of the macropore volume and of the mesopore volume) measured by mercury intrusion according to standard ASTM D4284-83, of between 0.20 cm³.g⁻¹ and 0.40 cm³.g⁻¹, and preferably between 0.20 cm³.g⁻¹ and 0.35 cm³.g⁻¹ (expressed relative to the anhydrous equivalent mass of the zeolite adsorbent).

8. The zeolite adsorbent according to any one of the preceding claims, having a ratio (macropore volume)/(macropore volume+mesopore volume) of between 0.2 and 1, preferably between 0.4 and 0.8 and even more preferably between 0.45 and 0.65, the macropore volume and the mesopore volume being measured by mercury intrusion according to standard ASTM D4284-83.

9. The use of at least one zeolite adsorbent according to any one of claims 1 to 8, for separating para-xylene from aromatic fractions containing 8 carbon atoms.

10. A process for separating para-xylene from aromatic C8 isomer fractions, **characterized in that** the process comprises a step of adsorption, in gas phase or liquid phase, which consists in using a zeolite adsorbent according to any one of claims 1 to 8.

11. The process for separating xylene according to claim 10, **characterized in that** the process comprises at least the following steps:
a) placing the feedstock in contact, under suitable adsorption conditions, with a bed of zeolite adsorbent, so as preferentially to adsorb the para-xylene,
b) placing the adsorbent bed in contact, under desorption conditions, with a desorbent,
c) removing from the adsorbent bed a stream containing the desorbent and the products of the feedstock that are the least selectively adsorbed,
d) removing from the adsorbent bed a stream containing the desorbent and the para-xylene,
e) separation of the stream obtained from step c) into a first stream containing the desorbent and a second stream containing the products of the feedstock that are the least selectively adsorbed, and
f) separation of the stream obtained from step d) into a first stream containing the desorbent and a second stream containing para-xylene at a level of purity greater than or equal to 75% .

12. The process for separating paraxylene according to claim 10 or 11, wherein the desorbent is selected from the group consisting of toluene and para-diethylbenzene.
